# EUROPEAN PATENT SPECIFICATION

(11) **EP 3 196 308 B1**
(45) Date of publication and mention of the grant of the patent: **14.08.2019**
(21) Application number: 17152059.6
(22) Date of filing: 18.01.2017
(51) Int. Cl.: C12N 15/63, C12N 15/79, G01N 33/00

(54) **REPORTER VECTOR FOR EVALUATING CHARACTERISTICS OF SUBJECT CELL, ASSAY KIT, PROCEDURE AND DEVICE**
REPORTERVEKTOR ZUR BEURTEILUNG DER EIGENSCHAFTEN EINER SUBJEKTZELLE, TEST-KIT, VERFAHREN UND VORRICHTUNG
VECTEUR-RAPPORTEUR DESTINÉ À ÉVALUER LES CARACTÉRISTIQUES D'UNE CELLULE DE SUJET, KIT DE DOSAGE, PROCÉDÉ ET DISPOSITIF

(30) Priority: 19.01.2016 JP 2016008125
(43) Date of publication of application: 26.07.2017
(73) Proprietor: Toshiba Medical Systems Corporation, Otawara-shi, Tochigi-ken 324-8550 (JP)
(72) Inventor: AKAHOSHI, Eiichi, Tokyo, 105-8001 (JP); NOZAKI, Emi, Tokyo, 105-8001 (JP); ISHIHARA, Mitsuko, Tokyo, 105-8001 (JP)
(74) Representative: Moreland, David

(56) References cited:
- WO-A1-2013/157020
- WO-A1-2014/185549
- JP-A- 2016 101 161
- MATTHEW MAHON: "Vectors bicistronically linking a gene of interest to the SV40 large T antigen in combination with the SV40 origin of replication enhance transient protein expression and luciferase reporter activity", BIOTECHNIQUES RAPID DISPATCHES, 1 August 2011 (2011-08-01), XP55295383, US ISSN: 0736-6205, DOI: 10.2144/000113720
- Anonymous: "pmirGLO Dual-Luciferase miRNA Target Expression Vector", promega.com , 1 August 2016 (2016-08-01), XP002770678, Retrieved from the Internet: URL:https://www.promega.com/-/media/files/ resources/protocols/product-information-sh eets/a/pmirglo-dual-luciferase-mirna-targe t-expression-vector-protocol.pdf [retrieved on 2017-05-31]
- HE JIEYU ET AL: "Nampt/Visfatin/PBEF: A functionally multi-faceted protein with a pivotal role in malignant tumors", CURRENT PHARMACEUTICAL DESIGN, vol. 18, no. 37, 1 December 2012 (2012-12-01), pages 6123-6132, XP055171101, NL ISSN: 1381-6128, DOI: 10.2174/138161212803582531

### SEQUENCE LISTING

<110> Toshiba Medical Systems Corporation
<120> REPORTER VECTOR FOR EVALUATING CHARACTERISTICS OF SUBJECT CELL, ASSAY KIT, PROCEDURE AND DEVICE
<130> 16S0308
<150> JP 2016-008125
   <151> 2016-01-19
<160> 11
<170> PatentIn version 3.5
<210> 1
   <211> 2702
   <212> DNA
   <213> Human
<400> 1
   ccatgttgcc caggctggtc tcaaaatcct gggctcaagt gatcagcccg cctcagcctc      60
   ccaaagtgct gggattacag gtgtgagcca ccacgtccaa ccttaatgct agctgttaaa     120
   ctgtctttca ggggaggcca ttttaaaatg aaatcttggc tctgttttca gtgataattt     180
   catagttcta aaccccaatt aattctgaat tgctccagct taagcatgta gttacataga     240
   gaatgtgcct atttctatct caggcaaatg gtgattgtcc tattggtaac atgacttcat     300
   tgtcattttt tttctctgga gactactgtg aggattcaca ttactaatac ttgtgcaggc     360
   ctcactcctt aatttaacaa gcaatgtcaa tacaatattt tttctttgca cagatataag     420
   acctttttat tttaaaactg caaattcaac tcaaagattg caagatcaaa ttttgcatct     480
   taaaaaaata ctaccataaa caaagcccac attataatca gaatcctact ttcttttctg     540
   cataaagcag catagaacaa cggtcattta tagatacatg aattccagga agctaaacaa     600
   ataagaattt gtactataag taaagtaggt tatataaatt ttaaatgaag gaaaaaacac     660
   caaattaagg acaaaaatta tctttttcct tctttgaatt gcactccttg aaataatata     720
   aaaaaactcc caaaatcaaa tattaaaaaa taatttgttt aggccgggcg cggtggctca     780
   cgtctgtaat cccagcactt tgggaggccg aggcgggagg atcacgaggt caggagatcg     840
   agaccatcct ggctaagacg gtgaaacccc atctctacta aaaatacaaa aaattagccg     900
   ggcgtggtgg cgggcgcctg tagtcccagc tgcttgggag actgaggcag gaggatggcg     960
   tgaaccaggg aggcggagct tgcagtgagc cgagatccag ccactgcact ccagcctggg    1020
   cgacagagcg agactccgtc tcaaaataga aataataatt tgtttaaatg cgtttacaaa    1080
   taattttccc cggtaaaaca cagggaagat caaccaataa atacataagc cctcttgttt    1140
   caaacctcgt tgctgaaaat aattgtaagt gtactagtat ttactacatt ggtgagtgcc    1200
   tggtgatacc ttccacttcc atgatctttg ccctgtattg tatctacgtg tagatgaggt    1260
   gattgtcaaa aatatatact gacttcaaga catagtgttg actgaggttg tctttattag      1320
   aagtctcagc catgcctcca gtttcgagat gaatctcaat tcacaagttc cactatgaat      1380
   ctcaattcac aagttccact accaccacca ccatcactgc ccccagatag aatgagactt      1440
   ctaataactt gaaagacaag tctataaata tcctgtaatc gttttcactg tatcactttt      1500
   tcctggctac ttttatccga taaaaacctc aatttatttt taagctatta aacatgcgga      1560
   caaagggaat cttagtttta tgcaaaaacg caaccacgca tgagaactgc gcaagtcttt      1620
   cttggaatgg tctgtatttg ggtgaagtca tcagacgtca ttacacttct ttattttggg      1680
   gttgccaact cgtttcccag gatttaaaga ctataacgat gataaaagtc agtgtcgcac      1740
   cctgtcaaag gcttggcccg ttgccttttc cttcccggca atactcggtt caattaggtc      1800
   ttgtcccctc attatctgtg aggactgaat tccacccccg cttttcaacg caggctcttt      1860
   gctcgggaaa agtcaaacca tctctcaaag gatcaaagag ctcagccata gacagagccg      1920
   ccggaggaaa gcggagtcgc tgcatcagat gaaaggggcc cctcagcctc actcctcacc      1980
   gcagctcctg ggatcttaaa gacagggtca ggaggatcag gagggacaag agggatggag      2040
   gcgaaaggct ggatccttaa tccaggccgg agacaaaggt gagtaactgc gcgtggtgga      2100
   cgggaagatg ggcagtagga ggctcctcgc cccctagccg ggtggcgtga cttcaggagg      2160
   acccagaaaa ccggccagga ctgggtagga cggaaggtcc caggcggggc gggcgaaccg      2220
   gctcctcgtg gcactggcaa agctgggaac gccccctcca ggccccgccc tcccgcgccc      2280
   gtgccgggaa ctcgaactta ggtaagcgcc cgggtcacgc gccggggctg ccccgctgcc      2340
   tccagcttcc cagagctggc gtctgggagg aagagcgcgt gcatcacgtc ctcctccccg      2400
   ccccgcctcc gcgcactgcg cagcgcgctg ccgcgccccg ccctttctcg gcccccggag      2460
   ggtgacgggg tgaaggcggg ggaaccgagg tggggagtcc gccagagctc ccagactgcg      2520
   agcacgcgag ccgccgcagc cgtcacccgc gccgcgtcac ggctcccggg cccgccctcc      2580
   tctgacccct cccctctctc cgtttccccc tctccccctc ctccgccgac cgagcagtga      2640
   cttaagcaac ggagcgcggt gaagctcatt tttctccttc ctcgcagccg cgccagggag      2700
   ct                                                                    2702
<210> 2
   <211> 222
   <212> DNA
   <213> Simian virus 40
<400> 2
   cagacatgat aagatacatt gatgagtttg gacaaaccac aactagaatg cagtgaaaaa 60
   aatgctttat ttgtgaaatt tgtgatgcta ttgctttatt tgtaaccatt ataagctgca 120
   ataaacaagt taacaacaac aattgcattc attttatgtt tcaggttcag ggggaggtgt 180
   gggaggtttt ttaaagcaag taaaacctct acaaatgtgg ta 222
<210> 3
   <211> 228
   <212> DNA
   <213> Bovine
<400> 3
   gtttaaaccc gctgatcagc ctcgactgtg ccttctagtt gccagccatc tgttgtttgc 60
   ccctcccccg tgccttcctt gaccctggaa ggtgccactc ccactgtcct ttcctaataa 120
   aatgaggaaa ttgcatcgca ttgtctgagt aggtgtcatt ctattctggg gggtggggtg 180
   gggcaggaca gcaaggggga ggattgggaa gacaatagca ggcatgct 228
<210> 4
   <211> 153
   <212> DNA
   <213> Artificial Sequence
<220>
   <223> poly(A)signal
<400> 4
   aataaaatat ctttattttc attacatctg tgtgttggtt ttttgtgtga atcgatagta 60
   ctaacatacg ctctccatca aaacaaaacg aaacaaaaca aactagcaaa ataggctgtc 120
   cccagtgcaa gtgcaggtgc cagaacattt ctc 153
<210> 5
   <211> 654
   <212> DNA
   <213> Cytomegalovirus
<400> 5
   cgatgtacgg gccagatata cgcgttgaca ttgattattg actagttatt aatagtaatc 60
   aattacgggg tcattagttc atagcccata tatggagttc cgcgttacat aacttacggt 120
   aaatggcccg cctggctgac cgcccaacga cccccgccca ttgacgtcaa taatgacgta 180
   tgttcccata gtaacgccaa tagggacttt ccattgacgt caatgggtgg agtatttacg 240
   gtaaactgcc cacttggcag tacatcaagt gtatcatatg ccaagtacgc cccctattga 300
   cgtcaatgac ggtaaatggc ccgcctggca ttatgcccag tacatgacct tatgggactt 360
   tcctacttgg cagtacatct acgtattagt catcgctatt accatggtga tgcggttttg 420
   gcagtacatc aatgggcgtg gatagcggtt tgactcacgg ggatttccaa gtctccaccc 480
   cattgacgtc aatgggagtt tgttttggca ccaaaatcaa cgggactttc caaaatgtcg 540
   taacaactcc gccccattga cgcaaatggg cggtaggcgt gtacggtggg aggtctatat 600
   aagcagagct ctctggctaa ctagagaacc cactgcttac tggcttatcg aaat 654
<210> 6
   <211> 752
   <212> DNA
   <213> Human herpesvirus
<400> 6
   aaatgagtct tcggacctcg cgggggccgc ttaagcggtg gttagggttt gtctgacgcg 60
   gggggagggg gaaggaacga aacactctca ttcggaggcg gctcggggtt tggtcttggt 120
   ggccacgggc acgcagaaga gcgccgcgat cctcttaagc acccccccgc cctccgtgga 180
   ggcgggggtt tggtcggcgg gtggtaactg gcgggccgct gactcgggcg ggtcgcgcgc 240
   cccagagtgt gaccttttcg gtctgctcgc agacccccgg gcggcgccgc cgcggcggcg 300
   acgggctcgc tgggtcctag gctccatggg gaccgtatac gtggacaggc tctggagcat 360
   ccgcacgact gcggtgatat taccggagac cttctgcggg acgagccggg tcacgcggct 420
   gacgcggagc gtccgttggg cgacaaacac caggacgggg cacaggtaca ctatcttgtc 480
   acccggaggc gcgagggact gcaggagctt cagggagtgg cgcagctgct tcatccccgt 540
   ggcccgttgc tcgcgtttgc tggcggtgtc cccggaagaa atatatttgc atgtctttag 600
   ttctatgatg acacaaaccc cgcccagcgt cttgtcattg gcgaattcga acacgcagat 660
   gcagtcgggg cggcgcggtc ccaggtccac ttcgcatatt aaggtgacgc gtgtggcctc 720
   gaacaccgag cgaccctgca gcgacccgct ta 752
<210> 7
   <211> 599
   <212> DNA
   <213> encephalomyocarditis virus
<400> 7
   cctctagcgg gatcaattcc gccccccccc cctaacgtta ctggccgaag ccgcttggaa      60
   taaggccggt gtgcgtttgt ctatatgtta ttttccacca tattgccgtc ttttggcaat     120
   gtgagggccc ggaaacctgg ccctgtcttc ttgacgagca ttcctagggg tctttcccct     180
   ctcgccaaag gaatgcaagg tctgttgaat gtcgtgaagg aagcagttcc tctggaagct     240
   tcttgaagac aaacaacgtc tgtagcgacc ctttgcaggc agcggaaccc cccacctggc     300
   gacaggtgcc tctgcggcca aaagccacgt gtataagata cacctgcaaa ggcggcacaa     360
   ccccagtgcc acgttgtgag ttggatagtt gtggaaagag tcaaatggct ctcctcaagc     420
   gtattcaaca aggggctgaa ggatgcccag aaggtacccc attgtatggg atctgatctg     480
   gggcctcggt gcacatgctt tacgtgtgtt tagtcgaggt taaaaaacgt ctaggccccc     540
   cgaaccacgg ggacgtggtt ttcctttgaa aaacacgata ataccatgat tgaacaaga      599
<210> 8
   <211> 2133
   <212> DNA
   <213> Simian virus 40
<400> 8
   atggataaag ttttaaacag agaggaatct ttgcagctaa tggaccttct aggtcttgaa 60
   aggagtgcct gggggaatat tcctctgatg agaaaggcat atttaaaaaa atgcaaggag 120
   tttcatcctg ataaaggagg agatgaagaa aaaatgaaga aaatgaatac tctgtacaag 180
   aaaatggaag atggagtaaa atatgctcat caacctgact ttggaggctt ctgggatgca 240
   actgagattc caacctatgg aactgatgaa tgggagcagt ggtggaatgc ctttaatgag 300
   gaaaacctgt tttgctcaga agaaatgcca tctagtgatg atgaggctac tgctgactct 360
   caacattcta ctcctccaaa aaagaagaga aaggtagaag accccaagga ctttccttca 420
   gaattgctaa gttttttgag tcatgctgtg tttagtaata gaactcttgc ttgctttgct 480
   atttacacca caaaggaaaa agctgcactg ctatacaaga aaattatgga aaaatattct 540
   gtaaccttta taagtaggca taacagttat aatcataaca tactgttttt tcttactcca 600
   cacaggcata gagtgtctgc tattaataac tatgctcaaa aattgtgtac ctttagcttt 660
   ttaatttgta aaggggttaa taaggaatat ttgatgtata gtgccttgac tagagatcca 720
   ttttctgtta ttgaggaaag tttgccaggt gggttaaagg agcatgattt taatccagaa 780
   gaagcagagg aaactaaaca agtgtcctgg aagcttgtaa cagagtatgc aatggaaaca 840
   aaatgtgatg atgtgttgtt attgcttggg atgtacttgg aatttcagta cagttttgaa 900
   atgtgtttaa aatgtattaa aaaagaacag cccagccact ataagtacca tgaaaagcat 960
   tatgcaaatg ctgctatatt tgctgacagc aaaaaccaaa aaaccatatg ccaacaggct 1020
   gttgatactg ttttagctaa aaagcgggtt gatagcctac aattaactag agaacaaatg 1080
   ttaacaaaca gatttaatga tcttttggat aggatggata taatgtttgg ttctacaggc 1140
   tctgctgaca tagaagaatg gatggctgga gttgcttggc tacactgttt gttgcccaaa 1200
   atggattcag tggtgtatga ctttttaaaa tgcatggtgt acaacattcc taaaaaaaga 1260
   tactggctgt ttaaaggacc aattgatagt ggtaaaacta cattagcagc tgctttgctt 1320
   gaattatgtg gggggaaagc tttaaatgtt aatttgccct tggacaggct gaactttgag 1380
   ctaggagtag ctattgacca gtttttagta gtttttgagg atgtaaaggg cactggaggg 1440
   gagtccagag atttgccttc aggtcaggga attaataacc tggacaattt aagggattat 1500
   ttggatggca gtgttaaggt aaacttagaa aagaaacacc taaataaaag aactcaaata 1560
   tttccccctg gaatagtcac catgaatgag tacagtgtgc ctaaaacact gcaggccaga 1620
   tttgtaaaac aaatagattt taggcccaga gattatttaa agcattgcct ggaacgcagt 1680
   gagtttttgt tagaaaagag aataattcaa agtggcattg ctttgcttct tatgttaatt 1740
   tggtacagac ctgtggctga gtttgctcaa agtattcaga gcagaattgt ggagtggaaa 1800
   gagagattgg acaaagagtt tagtttgtca gtgtatcaaa aaatgaagtt taatgtggct 1860
   atgggaattg gagttttaga ttggctaaga aacagtgatg atgatgatga agacagccag 1920
   gaaaatgctg ataaaaatga agatggtggg gagaagaaca tggaagactc agggcatgaa 1980
   acaggcattg attcacagtc ccaaggctca tttcaggccc ctcagtcctc acagtctgtt 2040
   catgatcata atcagccata ccacatttgt agaggtttta cttgctttaa aaaacctccc 2100
   acacctcccc ctgaacctga acctgaaaca taa 2133
<210> 9
   <211> 2133
   <212> DNA
   <213> Simian virus 40
<400> 9
   atggataaag ttttaaacag agaggaatct ttgcagctaa tggaccttct aggtcttgaa      60
   aggagtgcct gggggaatat tcctctgatg agaaaggcat atttaaaaaa atgcaaggag     120
   tttcatcctg ataaaggagg agatgaagaa aaaatgaaga aaatgaatac tctgtacaag     180
   aaaatggaag atggagtaaa atatgctcat caacctgact ttggaggctt ctgggatgca     240
   actgagattc caacctatgg aactgatgaa tgggagcagt ggtggaatgc ctttaatgag     300
   gaaaacctgt tttgctcaga agaaatgcca tctagtgatg atgaggctac tgctgactct     360
   caacattcta ctcctccaaa aaagaagaga aaggtagaag accccaagga ctttccttca     420
   gaattgctaa gttttttgag tcatgctgtg tttagtaata gaactcttgc ttgctttgct      480
   atttacacca caaaggaaaa agctgcactg ctatacaaga aaattatgga aaaatattct     540
   gtaaccttta taagtaggca taacagttat aatcataaca tactgttttt tcttactcca     600
   cacaggcata gagtgtctgc tattaataac tatgctcaaa aattgtgtac ctttagcttt     660
   ttaatttgta aaggggttaa taaggaatat ttgatgtata gtgccttgac tagagatcca     720
   ttttctgtta ttgaggaaag tttgccaggt gggttaaagg agcatgattt taatccagaa     780
   gaagcagagg aaactaaaca agtgtcctgg aagcttgtaa cagagtatgc aatggaaaca     840
   aaatgtgatg atgtgttgtt attgcttggg atgtacttgg aatttcagta cagttttgaa     900
   atgtgtttaa aatgtattaa aaaagaacag cccagccact ataagtacca tgaaaagcat     960
   tatgcaaatg ctgctatatt tgctgacagc aaaaaccaaa aaaccatatg ccaacaggct    1020
   gttgatactg ttttagctaa aaagcgggtt gatagcctac aattaactag agaacaaatg    1080
   ttaacaaaca gatttaatga tcttttggat aggatggata taatgtttgg ttctacaggc    1140
   tctgctgaca tagaagaatg gatggctgga gttgcttggc tacactgttt gttgcccaaa    1200
   atggagtcag tggtgtatga ctttttaaaa tgcatggtgt acaacattcc taaaaaaaga    1260
   tactggctgt ttaaaggacc aattgatagt ggtaaaacta cattagcagc tgctttgctt    1320
   gaattatgtg gggggaaagc tttaaatgtt aatttgccct tggacaggct gaactttgag 1380
   ctaggagtag ctattgacca gtttttagta gtttttgagg atgtaaaggg cactggaggg 1440
   gagtccagag atttgccttc aggtcaggga attaataacc tggacaattt aagggattat 1500
   ttggatggca gtgttaaggt aaacttagaa aagaaacacc taaataaaag aactcaaata 1560
   tttccccctg gaatagtcac catgaatgag tacagtgtgc ctaaaacact gcaggccaga 1620
   tttgtaaaac aaatagattt taggcccaga gattatttaa agcattgcct ggaacgcagt 1680
   gagtttttgt tagaaaagag aataattcaa agtggcattg ctttgcttct tatgttaatt 1740
   tggtacagac ctgtggctga gtttgctcaa agtattcaga gcagaattgt ggagtggaaa 1800
   gagagattgg acaaagagtt tagtttgtca gtgtatcaaa aaatgaagtt taatgtggct 1860
   atgggaattg gagttttaga ttggctaaga aacagtgatg atgatgatga agacagccag 1920
   gaaaatgctg ataaaaatga agatggtggg gagaagaaca tggaagactc agggcatgaa 1980
   acaggcattg attcacagtc ccaaggctca tttcaggccc ctcagtcctc acagtctgtt 2040
   catgatcata atcagccata ccacatttgt agaggtttta cttgctttaa aaaacctccc 2100
   acacctcccc ctgaacctga acctgaaaca taa 2133
<210> 10
   <211> 2133
   <212> DNA
   <213> Simian virus 40
<400> 10
   atggataaag ttttaaacag agaggaatct ttgcagctaa tggaccttct aggtcttgaa      60
   aggagtgcct gggggaatat tcctctgatg agaaaggcat atttaaaaaa atgcaaggag     120
   tttcatcctg ataaaggagg agatgaagaa aaaatgaaga aaatgaatac tctgtacaag     180
   aaaatggaag atggagtaaa atatgctcat caacctgact ttggaggctt ctgggatgca     240
   actgagattc caacctatgg aactgatgaa tgggagcagt ggtggaatgc ctttaatgag     300
   gaaaacctgt tttgctcaga agaaatgcca tctagtgatg atgaggctac tgctgactct     360
   caacattcta ctcctccaaa aaagaagaga aaggtagaag accccaagga ctttccttca     420
   gaattgctaa gttttttgag tcatgctgtg tttagtaata gaactcttgc ttgctttgct      480
   atttacacca caaaggaaaa agctgcactg ctatacaaga aaattatgga aaaatattct     540
   gtaaccttta taagtaggca taacagttat aatcataaca tactgttttt tcttactcca     600
   cacaggcata gagtgtctgc tattaataac tatgctcaaa aattgtgtac ctttagcttt     660
   ttaatttgta aaggggttaa taaggaatat ttgatgtata gtgccttgac tagagatcca     720
   ttttctgtta ttgaggaaag tttgccaggt gggttaaagg agcatgattt taatccagaa     780
   gaagcagagg aaactaaaca agtgtcctgg aagcttgtaa cagagtatgc aatggaaaca     840
   aaatgtgatg atgtgttgtt attgcttggg atgtacttgg aatttcagta cagttttgaa 900
   atgtgtttaa aatgtattaa aaaagaacag cccagccact ataagtacca tgaaaagcat 960
   tatgcaaatg ctgctatatt tgctgacagc aaaaaccaaa aaaccatatg ccaacaggct 1020
   gttgatactg ttttagctaa aaagcgggtt gatagcctac aattaactag agaacaaatg 1080
   ttaacaaaca gatttaatga tcttttggat aggatggata taatgtttgg ttctacaggc 1140
   tctgctgaca tagaagaatg gatggctgga gttgcttggc tacactgttt gttgcccaaa 1200
   atggagtcag tggtgtatga ctttttaaaa tgcatggtgt acaacattcc taaaaaaaga 1260
   tactggctgt ttaaaggacc aattgatagt ggtaaaacta cattagcagc tgctttgctt 1320
   gaattatgtg gggggaaagc tttaaatgtt aatttgccct tggacaggct gaactttgag 1380
   ctaggagtag ctattgacca gtttttagta gtttttgagg atgtaaaggg cactggaggg 1440
   gagtccagag atttgccttc aggtcaggga attaataacc tggacaattt aagggattat 1500
   ttggatggca gtgttaaggt aaacttagaa aagaaacacc taaataaaag aactcaaata 1560
   tttccccctg gaatagtcac catgaatgag tacagtgtgc ctaaaacact gcaggccaga 1620
   tttgtaaaac aaatagattt taggcccaaa gattatttaa agcattgcct ggaacgcagt 1680
   gagtttttgt tagaaaagag aataattcaa agtggcattg ctttgcttct tatgttaatt 1740
   tggtacagac ctgtggctga gtttgctcaa agtattcaga gcagaattgt ggagtggaaa 1800
   gagagattgg acaaagagtt tagtttgtca gtgtatcaaa aaatgaagtt taatgtggct 1860
   atgggaattg gagttttaga ttggctaaga aacagtgatg atgatgatga agacagccag 1920
   gaaaatgctg ataaaaatga agatggtggg gagaagaaca tggaagactc agggcatgaa 1980
   acaggcattg attcacagtc ccaaggctca tttcaggccc ctcagtcctc acagtctgtt 2040
   catgatcata atcagccata ccacatttgt agaggtttta cttgctttaa aaaacctccc 2100
   acacctcccc ctgaacctga acctgaaaca taa 2133
<210> 11
   <211> 165
   <212> DNA
   <213> Simian virus 40
<400> 11
   gctagcaata aaatatcttt attttcatta catctgtgtg ttggtttttt gtgtgaatcg 60
   atagtactaa catacgctct ccatcaaaac aaaacgaaac aaaacaaact agcaaaatag 120
   gctgtcccca gtgcaagtgc aggtgccaga acatttctcg ctagc 165

## Description

### FIELD

The invention relates generally to a reporter vector for evaluating characteristics of a subject cell, an assay kit and a method therefor. Also disclosed herein is a device for evaluating characteristics of a subject cell.

### BACKGROUND

The first cause of death in our country is cancer. Cancer is a group of diseases with a common feature of formation of a malignant tumor, and various tumors as many as over two hundreds types have been identified up to now. The common features of the malignant tumors includes uncontrollable reproduction of cells, potential of invading neighboring tissues and metastaticity to any tissues in the whole body.

In the treatment of cancer, for example, a malignant tumor, it is important to select an appropriate therapy according to the character of the tumor (namely, the characteristics of the oncocyte). Therefore, it is necessary to evaluate the characteristics of an oncocyte, i.e., the benign or malignancy of the tumor. In the case of a malignant tumor, it is preferable to be able to evaluate the aggression thereof, that is, for example, invasive and/or metastatic potential.

For example, when a change in expression of a specific gene exhibits a high correlation with the characteristics of an oncocyte, the gene can be used as a tumor marker. By detecting the tumor marker, the characteristics of the tumor relevant to a genetic expression can be distinguished. For example, if a specific tumor marker is detected in blood, other body fluids or extracted oncocyte, existence of oncocyte with a high correlation to the marker is suggested. A well-known example of the tumor markers is the gene Ki-67 used for evaluating the proliferativity of oncocyte.

There is a method of measuring the promoter activity of a tumor marker with use of a reporter gene. According to this method, a vector in which a promoter of the tumor marker and a reporter gene are connected together is introduced to a subject cell, and the signal from the reporter gene is detected or measured, based on which the cell characteristics of the subject cell are evaluated. One of such promoter assays is the valuation method which locates a sequence for self-replication of a vector in a downstream of a promoter reporter gene.

Jieyu et al. ("Nampt/Visfatin/PBEF: A functionally multi-faceted protein with a pivotal role in malignant tumors", Current Pharmaceutical Design, 2012, 18, p.6123-6132) discloses that NAMPT is a primary, rate-limiting enzyme involved in NAD+ biosynthesis, required for cell growth, survival, DNA replication and repair and energy metabolism, and is correlated to various malignant tumors.

WO 2014/185549 discloses a method of obtaining epigenetic information of a cell, comprising transcribing the state of modification of a specific sequence in the genome of the subject cell onto a corresponding sequence in a self-replicating reporter nucleic acid construct by substitution of a functional group during replication of the report nucleic acid construct in the nucleus of a subject cell. The presence or absence of a signal in the subject cell can be detected, thereby providing epigenetic information about the subject cell.

Mahon 2011 ("Vectors bicistronically linking a gene of interest to the SV40 large T antigen in combination with the SV40 origin of replication enhance transient protein expression and luciferase reporter activity", Biotechniques, 1 August 2011) discloses that the combination of the Simian Virus large T antigen (SVLT) and the internal ribosomal entry site (IRES) in a vector bearing the SV40 origin of replication (SV40 ori) generates a positive feedback loop resulting in enhanced expression.

"pmirGLO Dual-Luciferase miRNA Target Expression Vector" (promega.com, 1 August 2016) is a vector designed to quantitatively evaluate microRNA (miRNA) activity by inserting miRNA target sites 3' of the firefly luciferase gene (*luc2*). This vector comprises *luc2* as a primary reporter to monitor mRNA regulation and *Renilla* luciferase *(hRluc-neo)* acting as a control reporter for normalization and selection.

JP 2016101161 discloses a gene marker for examining the cellular property of test cells, comprising mRNA, a peptide of a protein of nicotinamide phosphoribosyl-group transferase (NAMPT) gene, or a gene marker that of a polynucleotide comprising the promoter sequence of NAMPT gene.
However, more excellent evaluation methods are in further demand.

### BRIEF DESCRIPTION OF THE DRAWINGS

FIG. 1 is a diagram showing an example of a reporter vector of an embodiment.
FIG. 2 is a diagram showing another example of a reporter vector disclosed herein.
FIG. 3 is a diagram showing still another example of a reporter vector of an embodiment.
FIG. 4 is a flowchart illustrating an example of the method of valuating characteristics of a subject cell according to the embodiment.
FIG. 5 is a diagram showing an example of a cell characteristics evaluation device.
FIG. 6 is a flowchart illustrating an example of the cell characteristics valuation method which employs a cell characteristics evaluation device as disclosed herein.
FIG. 7 is a diagram showing an example of a cell characteristics evaluation device disclosed herein.
FIG. 8 is a diagram showing a configuration of the vector used in an example.
FIG. 9 is a diagram illustrating results obtained in the example.
FIG. 10 is a diagram illustrating other results obtained in the example.
FIG. 11 is a diagram showing a configuration of the vector used in the example.
FIG. 12 is illustrating results obtained in the example.
FIG. 13 shows photomicrographs showing results obtained in the example.

### DETAILED DESCRIPTION

In general, according to one embodiment, a reporter vector comprises a first reporter gene expression unit, a second reporter gene expression unit and a replication initiation sequence. The first reporter gene expression unit includes a NAMPT gene promoter sequence, a first reporter gene operably linked to the promoter sequence, and a first transcription termination sequence existing downstream of the first reporter gene. The second reporter gene expression unit includes a promoter sequence exhibiting constitutive activity, a second reporter gene operably linked to the promoter sequence exhibiting constitutive activity, a bicistronic expression sequence existing downstream of the second reporter gene, a replication initiation protein gene existing downstream of the bicistronic expression sequence, and a second transcription termination sequence existing downstream of the bicistronic expression sequence. The replication initiation sequence binds to a replication initiation protein synthesized from the replication initiation protein gene, thereby initiating replication of the reporter vector. A first signal obtained from a first reporter protein expressed from the first reporter gene and a second signal obtained from a second reporter protein expressed from the second reporter gene are different from each other, and the transcription directions of the first reporter gene expression unit and the second reporter gene expression unit are the same.

Examples and embodiments will now be described with reference to accompanying drawings.

Disclosed herein is a reporter vector for evaluating the characteristics of a subject cell, an assay kit, a method and a device therefore.

The reporter vector may comprise, for example, at least two reporter genes, that is, a first reporter gene to be expressed by activation of a promoter sequence of a marker for cell characteristics, a second reporter gene to be expressed by activation of a promoter sequence exhibiting a constitutive activity, and a replication initiation unit for reproducing the vector itself within the subject cell into which the vector is introduced. The reporter vector may include a further reporter gene other than the first reporter gene or the second reporter gene. Thus, the reporter vector may be a self-replicating multi-reporter vector. A self-replicating multi-reporter vector may be, for example, a self-replicating dual reporter vector when including two reporter genes. Moreover, when three reporter genes are included, it may be a self-replicating triple reporter vector. However, the reporter vector is not limited to these.

By the evaluation of the characteristics of a subject cell, it is determined whether or not the conditions of the "status" or "characteristics" of the cell itself, for example, the inside or outside of the cell, or the "environment" surrounding the cell match the specific predetermined conditions. Such conditions may be judged based on whether or not there are indexes which indicate a sign of a specific disease, onset of a specific disease, the grade of progression of the onset of a specific disease and/or the severity of a specific disease. Such conditions may be those of a substance in the cell, whose content changes with the onset, existence or degree of progress of a disease, for example. Such conditions may be concrete indexes, for example, the existence of a specific gene, the expression of a specific gene, the pH value inside or outside the cell, information of the redox, the existence of a specific ion, the existence of an enzyme, the existence of an enzyme substrate, the existence of a specific substance, or the like, whether or not it exists, the degree of the quantitative value of the existence thereof, the distribution of the existence thereof, and/or the change in existential state, etc.

For example, the evaluation of the characteristics of a subject cell may be evaluating, in the case of evaluation of the characteristics of a cancer cell or oncocyte, the tumor characteristics of the subject cell, that is, for example, whether it is metastatic or invasive, or its degree, and whether the cell is normal or abnormal. When a subject cell is or may be abnormal, the evaluation of the characteristics of a subject cell may include evaluating the degree of the abnormality, or the degree of malignancy. The evaluation of the characteristics of an oncocyte may be evaluating, for example, whether or not the oncocyte is metastatic or invasive, the degree of the metastaticity of the oncocyte, the degree of malignancy or non-malignancy of the oncocyte, or the degree of the normality or abnormality of the oncocyte.

Note that the term "cancer cell", "oncocyte", "cancer" and "tumor" are used interchangeably in this specification.

### 1. Reporter Vector

The reporter vector may be, for example, a self-replicating multi-reporter vector for evaluating the characteristics of a subject cell.

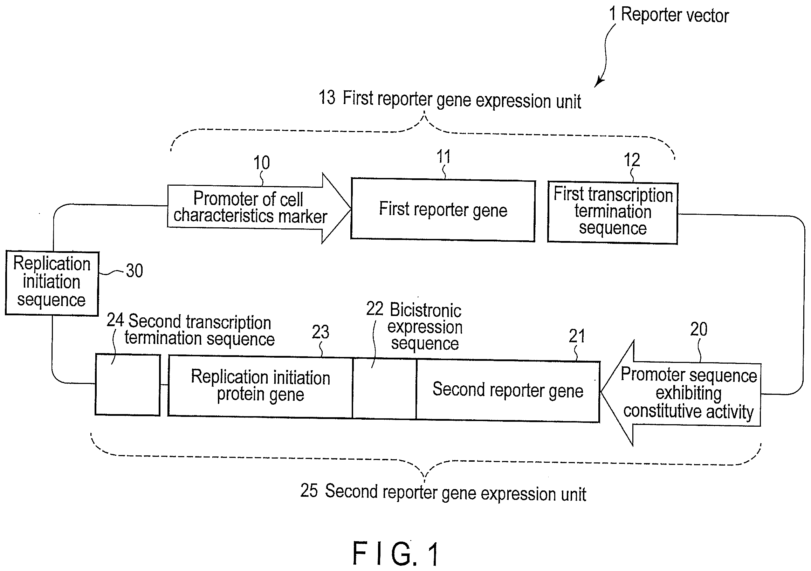

FIG. 1 shows a simplified example of the reporter vector. A reporter vector 1 comprises at least a first reporter gene expression unit 13, a second reporter gene expression unit 25 and a replication initiation unit.

The first reporter gene expression unit 13 includes a promoter sequence of a cell characteristics marker 10, a first reporter gene 11 locating downstream, and a first transcription termination sequence 12 locating further downstream. The promoter sequence of the cell characteristics marker 10, the first reporter gene 11, and the first transcription termination sequence 12 are functionally linked. Thus, the expression of the first reporter gene 11 is indicated by activation of the promoter sequence of the cell characteristics marker 10. These sequences will be described in detail later.

The second reporter gene expression unit 25 includes a promoter sequence exhibiting constitutive activity 20, a second reporter gene 21 locating downstream, a bicistronic expression sequence 22 locating further downstream, a replication initiation protein gene 23 locating further downstream, and a second transcription termination sequence 24 locating further downstream. The promoter sequence exhibiting constitutive activity 20, the second reporter gene 21, the bicistronic expression sequence 22, the replication initiation protein gene 23 and the second transcription termination sequence 24 are linked functionally. Thus, the second reporter gene 21 and the replication initiation protein gene 23 are expressed by activation of the promoter sequence exhibiting constitutive activity 20. These sequences will be described in detail later.

The replication initiation unit includes a replication initiation sequence 30 and a replication initiation protein gene 23 included in the second reporter gene expression unit. The replication initiation sequence 30 links to a replication initiation protein synthesized from the replication initiation protein gene 23, by which the replication of the self-replicating dual reporter vector 1 is started. The replication initiation sequence 30 may exist in any region of the sequence of the self-replicating dual reporter vector except the region where the first reporter gene expression unit 13 and the second reporter gene expression unit 25 exist.

Here, the expression "link functionally" is meant such a state that each nucleotide sequence included in a reporter vector links to be able to exhibit its target function. For example, in the case of a sequence encoding a protein, the amino acid sequences encoded by the base sequences are linked each other correctly. In other words, there is no misalignment between the frames of the amino acid codons, and a peptide which exhibits the target activity is synthesized in the cell into which the base sequence is introduced. Moreover, the expression "link functionally" is used interchangeably with "link operatably".

The first reporter gene expression unit 13, the second reporter gene expression unit 25 and the replication initiation sequence 30 are included on one reporter vector 1. The second reporter gene expression unit 25 may exist downstream the first reporter gene expression unit 13 and vice versa.

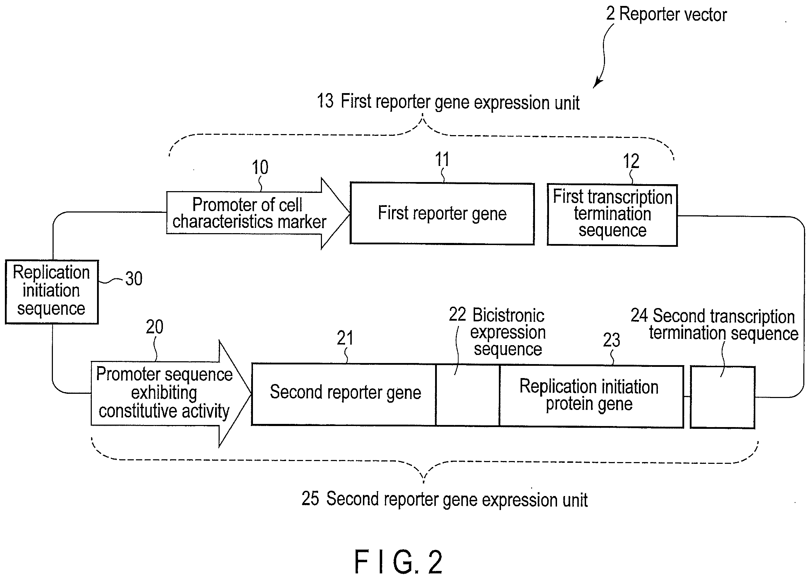

The transcription directions of the first reporter gene expression unit 13 and the second reporter gene expression unit 25 may be the same. But, the transcription directions of the first reporter gene expression unit 13 and the second reporter gene expression unit 25 may be in reverse to each other. An example of such a self-replicating dual reporter vector is shown in FIG. 2. This example a structure similar to that of the vector shown in FIG. 1 except the transcription directions of the second reporter gene expression unit 25 and the first reporter gene expression unit 13 are in reverse to each other.

The first reporter gene expression unit 13, the second reporter gene expression unit 25 and the replication initiation sequence 30 in the reporter vector may be arranged thereon clockwise in this order, that is, from 5' to 3', or may be in an opposite direction, but it is preferable to be arranged in the direction from 5' to 3'. Or the order may be the first reporter gene expression unit 13, the replication initiation sequence 30, and the second reporter gene expression unit 25.

The first reporter gene expression unit and the second reporter gene expression unit in the reporter vector may be arranged thereon clockwise in this order, that is, from 5' to 3', or may be in an opposite direction, but it is preferable that the two genetic expression units be arranged in the direction from 5' to 3'.

Although omitted from FIG. 1, between the first reporter gene expression unit 13, the second reporter gene expression unit 25 and the replication initiation sequence 30, arbitrary base sequences may be included unless the functions of these genetic expression units are damaged. Such base sequences may have specific functions or may be arbitrary base sequences without a particular function. The base sequences with specific functions may be, for example, a further reporter gene, a transcription termination sequence, a drug resistance gene, or a replication initiation sequence for colibacillus or yeast used for maintenance and regulation of the reporter vector, etc.

The further reporter gene may be a well-known reporter gene including a reporter gene similar to the first reporter gene or the second reporter gene, which will be described later. It is preferable that the signal obtained from the reporter protein expressed from the reporter gene and the signal obtained from the reporter protein expressed from the first or second reporter gene be identifiably different from each other.

By disposing the transcription termination sequence upstream the first reporter gene expression unit 13 and the second reporter gene expression unit 25, leak of a genetic expression from the first reporter gene expression unit 13 or the second reporter gene expression unit 25, and unexpected leak of the genetic expression from an arbitrary sequence on the plasmid can be decreased. Thus, the background (noise) caused by the leakage can be decreased, and the sensitivity and precision of the signal detection can be improved. The transcription termination sequence will be described in detail later.

Examples of the configuration elements of a reporter vector will be described.

### 1-1. First Reporter Gene Expression Unit

The promoter sequence of the cell characteristics marker 10 includes a promoter of the cell characteristics marker. The promoter of the cell characteristics marker may be a promoter of the cell characteristics marker originated from the subject cell. The cell characteristics marker is a gene which is expressed in the cell suffered from a specific disease, or is expressed more greatly or less in the cell suffered from a specific disease as compared with a normal cell. The cell characteristics marker to be expressed in a cell suffered from a specific disease may be, for example, a gene to be expressed only in the cell suffered from the specific disease. Each of these genes is expressed by activation of a promoter included therein. Thus, the activation of the promoter of a cell characteristics marker corresponds to the characteristics of the subject cell, that is, for example, the amount of expression of the cell characteristics marker according to the state of the cell itself or the environment surrounding the cell.

When the cell characteristics to be evaluated are tumor characteristics, the promoter sequence of the cell characteristics marker 10 may be a tumor marker. The tumor marker may be, for example, a tumor marker well-known by itself. The promoter of such a cell characteristics marker may include a gene promoter of, for example, NAMPT, Ki67, fos, myc or the like. The type of tumor to be detected may be, for example, epithelial tumor, non-epithelial tumor, hemopoietic tissue tumor, fetal tumor or any of combinations of these. The epithelial tumor may be, for example a mammary tumor or a lung tumor.

When the object to be detected is a cell relating to bone dybolism such as arthritis and osteoporosis, for example, a gene promoter of NFATC1 or CICC4, may be used. Or the object to be detected is a cell exposed to the oxidant stress, which may elicite various diseases, for example, a gene promoter of catalase or SOD may be used.

The promoter sequence of the cell characteristics marker 10 may be a polynucleotide including such a promoter as described above. The promoter sequence of the cell characteristics marker 10 may include a linker sequence in addition to the above-described promoters.

The promoter sequence of the cell characteristics marker 10 may be, for example, the sequence of the promoter of NAMPT gene represented by SEQ ID NO:1 shown in TABLE 1.

**TABLE 1**

| |
|---|
| < SEQ ID NO. 1> human NAMPT gene promoter |
| |

In some instances, the promoter sequence of the cell characteristics marker 10 may include a plurality of types of promoters. For example, the promoter sequence of the cell characteristics marker 10 may include a promoter of another gene in addition to the sequence of the promoter of the NAMPT gene.

The first reporter gene 11 may be any gene which encodes a reporter protein producing a detectable signal and can express a reporter protein by activation of the promoter sequence of the cell characteristics marker 10.

The detectable signal may be fluorescence, photogenesis, coloration or the like, or may be presentation of a substance such as protein. Or it may only be a signal detectable by, for example, a well-known method itself. The term "photogenesis" used here refers to chemoluminescence, bioluminescence or biochemical photogenesis.

The detectable signal may be a signal emitted from a reporter protein itself. Or, the signal concerned may be emitted from a reporter protein or any substance resulting from the reaction of the reporter protein and substances, for example, an enzyme reaction. Or, the signal concerned may be a signal produced from a reporter protein or any substance resulting from the binding of the reporter protein and the substances.

The reporter protein may be, for example, blue fluorescent protein, green fluorescent protein, red fluorescent protein, luciferase, β-galactosidase, nitric-monooxide synthetic enzyme, xanthine oxidase, or a heavy metal-binding protein.

A reporter gene which produces a signal from the reporter protein itself may be, for example, a gene which encodes a fluorescent protein such as a blue fluorescent protein, green fluorescent protein or red fluorescent protein.

The reporter gene which produces a signal from the reaction of a reporter protein and any substance may be, for example, a gene encoding a luminescent enzyme protein such as a firefly luciferase gene, a renilla luciferase gene or NanoLuc (registered trademark) luciferase gene, or a gene encoding an active oxygen generation enzyme such as a xanthine-oxidase gene or a nitric-monooxide synthetase gene, or a gene encoding a chromogenic enzyme protein such as a β-galactosidase gene or a chloramphenicol acetyltransferase gene.

The gene which produces a signal from the binding of a reporter protein and any substance may be a gene encoding a heavy metal binding protein. An example of the gene expresses a heavy metal binding protein as a detectable signal is disclosed in JP 2012-200245 A.

It is more preferable that the first reporter gene 11 be a gene encoding a luminescent enzyme protein such as a luciferase gene and a β-galactosidase gene.

The first transcription termination sequence 12 is a sequence for terminating the transcription of the gene upstream. The first transcription termination sequence 12 may be a sequence including, for example, a poly(A) sequence, and may be, for example, a poly(A) addition signal sequence.

The poly(A) addition signal sequence may be, for example, a poly(A) addition signal sequence of simian virus (SV) 40, a poly(A) addition signal sequence of a bovine growth hormone gene, or an artificially synthesized poly(A) addition signal. The poly(A) addition signal of SV40 virus, poly(A) addition signal of bovine growth hormone gene, or artificially synthesized poly(A) addition signal sequence may include, for example, base sequences represented by SEQ ID NO: 2, SEQ ID NO 3, and SEQ ID NO 4 provided in TABLE 2, respectively. Note that the poly(A) addition signal sequences usable in the embodiment are not limited to these, but unless the functions as the poly(A) addition signal sequences are impaired, those with a modified gene sequence may be used.

**TABLE 2**

| |
|---|
| < SEQ ID NO. 2> SV40 virus · poly(A) additional signal |
| |
| < SEQ ID NO. 3> Bovine growth hormon gene · poly(A) additional signal |
| |
| < SEQ ID NO. 4> artificial poly(A)additional signal |
| |

### 1-2. Second Reporter Gene Expression Unit

A promoter sequence exhibiting constitutive activity 20 contains a promoter exhibiting constitutive activity in a subject cell. Such a promoter may be activated when the transcription of the gene constitutively expressed is initiated within the subject cell.

The promoter sequence exhibiting constitutive activity 20 may include a linker sequence and the like in addition to the sequence having promoter activity.

The promoter sequence exhibiting constitutive activity 20 may be, for example, cytomegalovirus (CMV) promoter, thymidine kinase (tk) promoter of human herpesvirus, a promoter of a ubiquitin gene family origin or a polypeptide chain elongation factor (EF1α) gene promoter. The CMV promoter and tk promoter of human herpesvirus may include, for example, sequences represented by SEQ ID NO 5 and SEQ ID NO 6 provided in TABLE 3, respectively.

**TABLE 3**

| |
|---|
| < SEQ ID NO. 5> CMV promoter |
| |
| < SEQ ID NO. 6> human herpesvirus · tk promoter |
| |

A second reporter genes 21 may be similar to the first reporter gene 11 except that it can express a reporter protein by activation of the promoter sequence exhibiting constitutive activity 20. It is preferable that the signal obtained from the reporter protein expressed from the first reporter gene and the signal obtained from the reporter protein expressed from the second reporter gene 21 be identifiably different from each other.

A bicistronic expression sequence 22 may be a base sequence including a sequence which enables bicistronic expression in the subject cell. It can be achieved by binding ribosome to the inside of mRNA to initiate the translation of the protein without depending on 5' terminal cap structure of mRNA. The bicistronic expression means translating two types of proteins encoded by two genes existing upstream and downstream the bicistronic expression sequence 22 with one mRNA and express them simultaneously.

Therefore, with the bicistronic expression sequence 22 existing between the second reporter gene 21 and the replication initiation protein gene 23, the expressions of the second reporter gene 21 and the replication initiation protein gene 23 can be simultaneously controlled by the activation of the promoter sequence exhibiting constitutive activity 20.

The bicistronic expression sequence 22 may be, for example, internal ribosomal entry site (IRES) sequence. The IRES sequence may be of encephalomyocarditis virus or the like. The IRES sequence of the encephalomyocarditis virus may include, for example, a base sequence represented by SEQ ID NO: 7 provided in TABLE 4.

**TABLE 4**

| |
|---|
| < SEQ ID NO. 7> encephalomyocarditis virus mRNA internal ribosomal entry site (IRES) |
| |

The replication initiation protein gene 22 may be a gene encoding a protein which initiates the self-replication of the reporter vector 1 in a subject cell. The replication initiation protein gene 22 may be, for example, a wild type large T-antigen gene of simian virus 40, EBNA-1 gene of an Epstein-Barr virus, or a large T-antigen gene of murine polyomavirus. The replication initiation protein gene 22 may be, for example, a base sequence represented by SEQ ID NO: 8 provided in TABLE 5, or SEQ ID NO: 9 provided in TABLE 6, or SEQ ID NO: 10 provided in TABLE 7. The replication initiation protein gene 22 may be a nucleic acid having a DNA replication initiation function, represented by a sequence of SEQ ID NO: 8, SEQ ID NO: 9 or SEQ ID NO: 10 in which one or several bases are deleted, substituted or added. Here, the DNA replication initiation function is defined by encoding a protein which can initiate replication.

A second transcription termination sequence 24 may be a sequence for terminating the transcription of the second reporter gene 21 and the replication initiation protein gene 23 gene which may exist upstream, and may be a sequence similar to that of the first transcription termination sequence 12.

**TABLE 5**

| |
|---|
| < SEQ ID NO. 8> SV40 large T-antigen gene (wild type) |
| |

**TABLE 6**

| |
|---|
| < SEQ ID NO. 9> SV40 large T-antigen gene (variant 1) |
| |

**TABLE 7**

| |
|---|
| < SEQ ID NO. 10> SV40 large T-antigen gene (variant 2) |
| |

### 1-3. Replication Initiation Sequence

A replication initiation sequence 30 may be a base sequence including a sequence which initiates the replication of the reporter vector 1 by binding replication initiation protein thereto. The replication initiation protein may be a replication initiation protein expressed from the replication initiation protein gene 23, or a replication initiation protein existing in a subject cell.

The replication initiation sequence 30 can be selected appropriately based on the origin organism of the replication initiation protein gene 23, the type of the replication initiation protein expressed thereby, etc.

For example, when the replication initiation protein gene 23 is a large T-antigen gene of simian virus 40, a replication initiation sequence from the simian virus 40 may be used as the replication initiation sequence 30. In that case, the replication initiation sequence 30 may include a base sequence of SEQ ID NO: 11 provided in TABLE 8.

When the replication initiation protein gene 23 is EBNA-1 gene of Epstein-Barr virus, a replication initiation sequence from the Epstein-Barr virus may be used as the replication initiation sequence 30.

When the replication initiation protein gene 23 is a large T-antigen gene of murine polyomavirus, a replication initiation sequence from the murine polyomavirus may be used as the replication initiation sequence 30.

**TABLE 8**

| |
|---|
| < SEQ ID NO. 11> SV40 replication initiation sequence |
| |

Although these examples are preferable, they are not limiting.

With the above-described configuration, the first signal from the first reporter gene according to the quantity of the cell characteristics marker in the subject cell, and the second signal from the second reporter gene according to the quantity of the gene having the constitutive activity are simultaneously obtained from the same subject cell. For example, the second signal can be used for standardization of the first signal. For example, the relative value of the first signal may be computed by dividing the first signal by the second signal. Thus, the dispersion in the amount of the reporter vector introduced by the subject cell can be corrected, thereby making it possible to estimate the cell characteristics of the subject cell with high precision.

By being expressed the replication initiation protein gene by the promoter with constitutive activity, the reporter vector itself is cumulatively replicated. Thus, the first signal and the second signal can be amplified. Consequently, the cell characteristics of a subject cell can be estimated with precision higher than those of the conventional techniques.

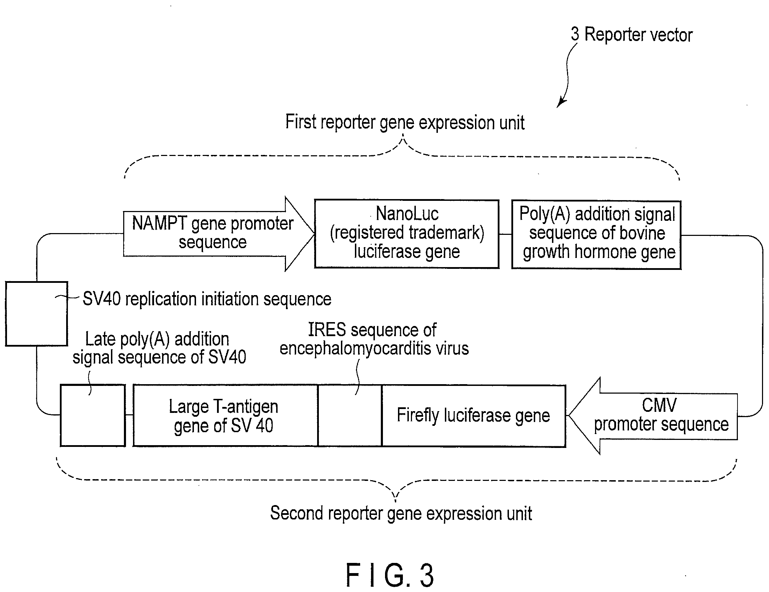

A further example of the reporter vector of the invention is shown in FIG. 3.

A reporter vector 3 includes a first reporter gene expression unit, a second reporter gene expression unit and a replication initiation sequence. The promoter of a cell characteristics marker included in the first reporter gene expression unit is a promoter of the NAMPT gene represented by SEQ ID NO: 1. The first reporter gene is NanoLuc (registered trademark) luciferase gene, and the first transcription termination sequence is a poly(A) addition signal sequence of bovine growth hormone gene, represented by SEQ ID NO: 3.

The promoter sequence exhibiting the constitutive activity included in the second reporter gene expression unit is a CMV promoter represented by SEQ ID NO: 5. The second reporter gene is a firefly luciferase gene and the bicistronic expression sequence is the IRES sequence of the encephalomyocarditis virus represented by SEQ ID NO 7. The replication initiation protein gene is a large T-antigen gene of SV40 represented by SEQ ID NO 10, and the transcription termination sequence is a late poly(A) addition signal sequence of SV40, represented by SEQ ID NO 2.

Moreover, the replication initiation sequence is a replication initiation sequence of SV40, represented by SEQ ID NO 11.

Note that the reporter vector is not limited to those described above.

### 2. Assay Kit for Evaluating Cell Characteristics of Subject Cell

In another aspect, there is provided an assay kit for evaluating the cell characteristics of a subject cell which contains the reporter vector of the above embodiment can be provided.

The assay kit contains at least one kind of reporter vector of those discussed above in Section 1.

The assay kit may contain a carrier which supports a reporter vector, and/or any reagent necessary for evaluating the characteristics of the subject cell.

The carrier may be, for example, a buffer solution, liposome, cationic lipid, cationic polymer, calcium chloride or nanoparticle of apatite.

The reagent may be, for example, an excipient, a stabilizer, a diluent and/or an auxiliary material, etc.

The assay kit may contain a substance necessary for detecting of a signal selected according to the types of the first and/or second reporter genes of the reporter vector included in the assay kit.

The substance necessary for detecting of the signal may be a substance which produces a signal when reacting with a reporter protein (for example, substrate), an additive which detects the substance produced by the reaction, or a substance which produces a signal when binding to a reporter protein.

The reporter vector, carrier and reagent can be contained each separately or in any combination in a container or containers, to be provided.

### 3. Method of Evaluate Cell Characteristics of Subject Cell

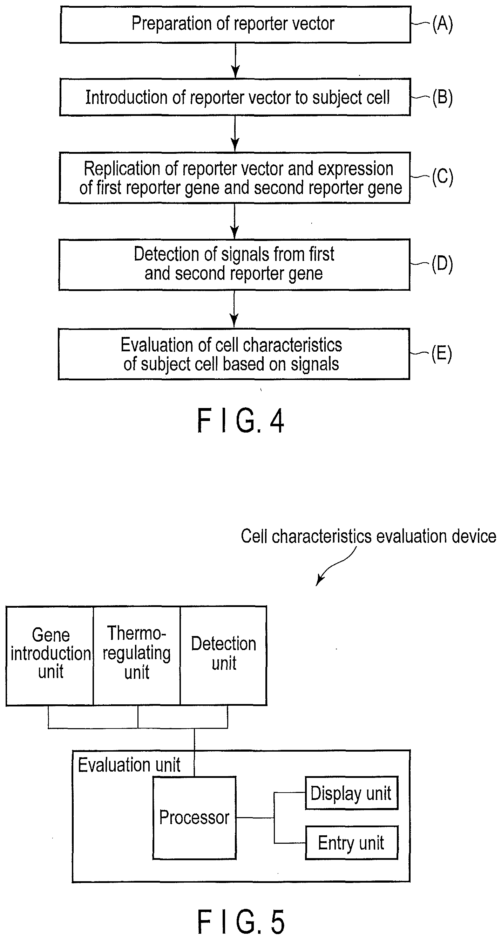

A method of evaluating the characteristics of a subject cell using the above-described reporter vector may comprise the following steps shown in FIG. 4.
(A) preparing a reporter vector;
(B) introducing the reporter vector into the subject cell;
(C) replicating the reporter vector to express a first reporter gene and a second reporter gene;
(D) detecting a first signal from the first reporter gene and a second signal from the second reporter gene; and
(E) evaluating the cell characteristics of the subject cell based on the first signal and the second signal.

The steps will now be further described.

### (A) Preparation of Reporter Vector

The reporter vector can be produced and prepared by integrating a first reporter gene expression unit, a second reporter gene expression unit and a replication initiation sequence on the same vector.

The vector may be any conventionally known vector in this technical field, for example, a plasmid, a cosmid or a phage. A plasmid vector is preferable.

The vector may be produced by any method known to a person skilled in the art. The vector may be prepared in the state of being contained in a carrier, which will be described later.

### (B) Introduction of Reporter Vector to Subject Cell

The reporter vector obtained in the step (A) may be introduced to a subject cell and/or a cell used as a standard.

The subject cell may be extracted from a subject. The subject may be, for example, any of mammalians including primates such as apes and humans, rodents such as mice and rats, companion animals such as rabbits, dogs and cats and livestock including horses and cows.

The subject cell should sufficiently be a cell whose characteristics are to be evaluated. The subject cell may be a cell colony or a single cell, or may be contained in a sample. The sample may be, for example, blood, urine, feces, saliva, mucosa in the oral cavity, coelomic fluid, expectoration, or a tissues obtained by an biopsy or the like.

The cell characteristics to be evaluated as to the subject cell is canceration of the cell, and when the object of detection is a cancer cell, the subject cell may be, for example, an epithelial tumor, non-epithelial tumor, hemopoietic tissue tumor, fetal tumor, or any combination of these. The epithelial tumor may be, for example, a mammary tumor or a lung tumor.

The reporter vector may be introduced by any conventionally known method.

### (C) Replication of Reporter Vector and Expression of Reporter Vector

After introducing the reporter gene into the subject cell, the subject cell may be maintained under the conditions that the vector can be self-replicated and the first and second reporter genes can be expressed. The conditions may be culture conditions conventionally known to a person skilled in the art, in which a subject cell is divided to be reproduced.

Under such conditions, the first and second reporter genes may be expressed. At the same time, the reporter vector can self-replicate as the replication initiation protein expressed by the activation of the promoter sequence exhibiting constitutive activity binds to the replication initiation sequence on the reporter vector. As the first and second reporter genes on the reporter vector cumulatively self-replicating are expressed, the first signal and the second signal can be amplified.

### (D) Detection of Signals from Reporter genes

The first and second signals may be detected, for example, by detecting or quantifying an optical signal obtained from the reporter proteins synthesized from the first and second reporter genes. The detection method may be selected appropriately according to the type of reporter protein encoded by the reporter gene. The method will be described below.

When the reporter gene is luciferase gene, a luciferase protein is extracted from the subject cell, and luciferin, which is a kind of the substrate, is added thereto, to cause a photogenesis reaction, and then the luminescence intensity of the solution may be detected as a signal using a luminance measuring device, for example, a luminometer.

When the reporter gene is a fluorescent protein gene, for example, an extract containing the subject cell or the fluorescent protein extracted from the subject cell is irradiated with the light of a specific wavelength, and the intensity of the fluorescence generated from the fluorescent protein in the subject cell may be detected as a signal using a fluorometry device.

When the reporter gene is a β-galactosidase gene, β-galactosidase protein is extracted and a substrate such as 5-bromo-4-chloro-3-indolyl-β-D-galactopyranoside (X-Gal) or o-nitrophenyl-β-D-galactopyranoside (ONPG) is added to the solution, and then the absorbance thereof may be measured as a signal using a spectrometry device.

When the reporter gene is a nitric-monooxide-synthetase gene or xanthine-oxidase gene, the nitric-monooxide-synthetase or xanthine oxidase is extracted and the substrate is added to cause generation of active oxygen, and then the presence/absence or quantity thereof may be detected as a signal with an electron spin resonance device (ESR device) or the like. With the method using an ESR device, the quantity of active oxygen generated may be measured directly, or may be measured using a specific spin trap agent. When using a spin trap agent, the active oxygen generation enzyme is first trapped with the spin trap agent, and then the quantity of the active oxygen generated from the trapped active oxygen generation enzyme may be measured.

When the reporter gene is a heavy-metal binding protein gene, the quantity of the protein bound to the heavy metal originated from the reporter protein may be detected as a signal with a magnetic resonance imaging device, a nuclear-medicine diagnostic device, an MRI imaging device, or computerized transverse axial tomography. The quantity of the heavy-metal binding protein may be detected, for example, as follows. First, a measurable heavy metal is added in advance to the culture medium of the subject cell. Then, after washing the subject cell as needed, the heavy-metal binding protein is extracted from the subject cell. Next, the extracted heavy metal binding protein is imaged with a diagnostic imaging device applicable to the heavy metal added to the medium, and the quantity of the heavy metal binding protein is measured. The heavy-metal binding protein may be expressed inside the subject cell or on an outer surface of the subject cell.

The heavy-metal binding protein gene may be, for example, a base sequence encoding a metallic compound binding peptide. For example, the metallic compound binding protein may just be a peptide, oligopeptide, polypeptide and/or protein binding specifically to a specific metallic compound.

For example, the sequence encoding a metallic compound binding peptide may be a base sequence of an antibody gene or a single-strand antibody gene known to bind a desired metallic compound, and may just be designed based on such a base sequence. Such a base sequence may be designed, for example, by modification and/or changing such as substitution, deletion and addition of one or some bases within a range that can maintain the binding with the metallic compound, or by such modification and/or changing according to the object to be applied.

The gene encoding a single-strand antibody peptide can be designed from an amino acid sequence of the antibody to which the metallic compound binds.

For example, in an MRI imaging, a gadolinium compound is used preferably because of its high contrasting effect. The gadolinium compound should just be, for example, a metallic compound of gadolinium, gadolinium ion, gadolinium complex, a salt or derivative of any of these, a derivative containing any of these, or an analogue of a gadolinium compound.

Moreover, the heavy-metal binding protein gene may be a reporter gene exhibiting a metallic compound binding capacity extracellularly. Such a reporter gene may be a base sequence which encodes a metallic compound binding peptide exhibited extracellularly. Such a base sequence should only be configured, for example, so that it is transcribed and translated in the cell to produce a metallic compound binding peptide and then the produced metallic compound binding peptide moves to the cell membrane to exhibit the metallic compound binding capacity extracellularly. An example of such a reporter gene is disclosed in JP 2012-200245 A, for example. As disclosed therein, such a reporter gene should only comprise a base sequence encoding a metallic compound binding peptide, a base sequence encoding a signal peptide which conveys the metallic compound binding peptide to a cell membrane, and a base sequence encoding an anchor peptide which immobilizes the metallic compound binding peptide conveyed to the cell membrane by the signal peptide thereto.

The result obtained by detection of a signal may be the presence/absence of the signal, may be the size of the signal, or both.

When detecting the first signal, the second signal may be detected after abolishing the first signal, or the order may be reversed. Or the first signal and the second signal may be separated from each other with an appropriate filter and then detected simultaneously.

### (E) Evaluation of Characteristics of Subject Cell Based on First Signal and Second Signal

The value of the first signal acquired in the steps of (D) above may be divided by the second signal A to calculate out a relative value. The relative value may be compared between the subject cell and a cell used as a standard.

The comparison may be carried out by performing the steps of (A) to (E) on the subject cell and the standard cell simultaneously to calculate out the relative values to be compared. Or the relative value of a cell used as the standard may be computed in advance, to be compared therewith.

In the above-provided descriptions, a method including preparation of a reporter vector as step (A) is discussed, but the method may not necessarily include preparation of a reporter vector. The method may be carried out using a reporter vector prepared in advance.

With the above-described method of evaluating the characteristics of a subject cell, it is possible to evaluate the cell characteristics more precisely.

### 4. Device of Evaluation of Subject Cell

The above-described method of evaluating the characteristics of a subject cell can be carried out with a cell characteristics evaluation device. The cell characteristics evaluation device will be described with reference to FIG. 5. FIG. 5 is a block diagram showing an outline of the cell characteristics evaluation device. The cell characteristics evaluation device is one example of the device which detects the characteristics of a subject cell and evaluates the characteristics of the subject cell based thereon.

The cell characteristics evaluation device comprises the following structural elements:
(i) a gene introduction unit which introduces a reporter vector into a subject cell;
(ii) a thermo-regulating unit which receives a sample containing the subject cell obtained in the gene introduction unit and cultures the subject cell for a desired time;
(iii) a detection unit which receives the sample from the thermo-regulating unit and detects signals produced from the first reporter gene and the second reporter gene of the subject cell contained in the sample; and
(iv) an evaluation unit which evaluates cell characteristics of the subject cell based on a result of the detection.

In the gene introduction unit, the reporter vector is introduced into a nucleus of the subject cell. The reporter vector may be any reporter vector discussed above.

In the thermo-regulating unit, the self-replicating vector self-replicates within the nucleus of the subject cell.

In the detection unit, the signals produced from the first reporter gene and the second reporter gene are detected. In the detection unit, the subject cell is subjected to, for example, photogenesis measurement, fluorometry, absorbance measurement, X-ray measurement, electron spin resonance, nuclear medicine diagnosis and/or magnetic resonance imaging, etc. The measuring devices for these measurements may be selected according to the first reporter gene and the second reporter gene. The information acquired by the detection unit is sent to the evaluation unit.

The evaluation unit may comprise a processor, a display unit, an entry unit, etc. In the evaluation unit, the information acquired by the detection unit may be subjected to data processing by the processor and displayed on the display unit as needed. That is, the result obtained by the detection unit may be sent to the processor of the evaluation unit, where it may be analyzed, calculated and processed according to the predetermined procedures, and may be indicated on the display unit.

The insides of the gene introduction unit, the thermo regulating unit and the detection unit may be communicated to each other via connection windows so that processing can be continuously carried out. After the subject cell is passed to one unit to the following unit, the respective connection window can be closed by a closure shield.

The cell characteristics evaluation device may further include a cell separation unit (not shown) configured to separate a subject cell from a sample and send the subject cell to the gene introduction unit.

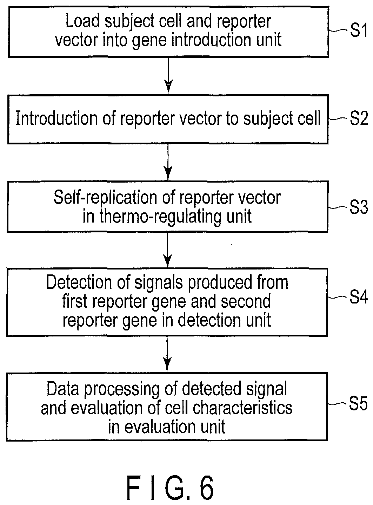

The procedure of the method of evaluating cell characteristics with the cell characteristics evaluation device will be described with reference to FIG. 6.

First, an experimenter may place a container containing a subject cell and a reporter vector into the gene introduction unit (S1). Then, the instruction to start the analysis may be entered from the entry unit. The instruction from the entry unit may be sent to the processor and with an instruction from the processor, a treatment to enabling the introduction of the reporter vector to the subject cell may be performed in the gene introduction unit (S2). After a predetermined time elapses, the introduction process may be finished and the subject cell accommodated in the container may be sent to the thermo-regulating unit. In the thermo-regulating unit, the subject cell may be maintained under predetermined conditions for a predetermined time. Thus, the reporter vector may self-replicate (S3). After the predetermined time elapses, the subject cell accommodated in the container may be sent to the detection unit from the thermo-regulating unit. In the detection unit, the signals produced from the first reporter gene and the second reporter gene of the subject cell may be detected (S4). The detected information may be sent to the evaluation unit to be subjected to data processing by the processor, and the cell characteristics may be evaluated based on the processed data (S5). The information thus obtained may be shown in the display unit. The container may be moved to the gene introduction unit, the thermo-regulating unit and the detection unit by a transportation means such as a conveyor belt, a movable arm and/or a movable tray, etc. All of these movements may be performed under control of the processor according to the directions of the processor based on a preset program. The gene introduction unit has a configuration required for the gene introduction by a selected method. The thermo-regulating unit has a configuration required in order to fulfill conditions required for the reporter vector to self-replicate. The detection unit has a configuration required for detection the signal according to the signal detected.

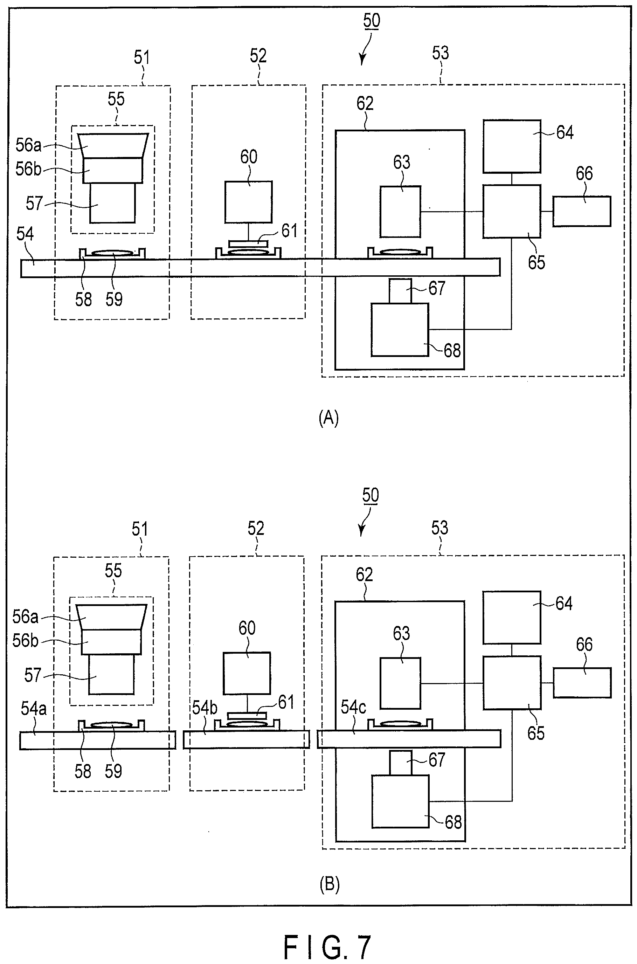

FIG. 7 shows an example of such a cell characteristics evaluation device.

A cell characteristics evaluation device 50 shown in FIG. 7(A) may include a cell separation unit 51 for cell separation, a DNA introduction unit 52 for gene introduction, a measurement unit 53 for measurement and evaluation, and a stage 54 placed continuously to follow the above members. The cell separation unit 51, the DNA introduction unit 52 and the measurement unit 53 may share the stage 54 to communicate each other by the stage 54. In other words, the cell separation unit 51, the DNA introduction unit 52 and the measurement unit 53 are arranged in this order from upstream to downstream above the stage 54.

The cell separation unit 51 may include a separator 55 for separating a cell to be examined, i.e., a subject cell, from a specimen obtained from an object. The separator 55 may comprise a receptor 56a for receiving the specimen obtained from the object, a treatment unit 56b which, for example, digest the specimen received with the receptor 56a, and a separation portion 57 for separating the subject cell from the specimen treated with the treatment unit 56b. The receptor 56a, the treatment unit 56b, and the separation portion 57 may be stacked one on another in this order as one liquid-tight integral body while the three hollow cylindrical portions having different internal diameters are connected together from the top to the bottom. The respective boundaries between the receptor 56a, the treatment unit 56b, and the separation portion 57 may be joined in a liquid junction manner with valves to be openable/closable. The receptor 56a may be a tapered cylindrical member with an aperture in its upper part and may comprise an openable/closable cover. The boundary between the receptor 56a and the treatment unit 56b is closed with a valve to be liquid-tight. When this valve is closed, the valve serves as a bottom of the receptor 56a. The treatment unit 56b is a cylindrical member continuing from the lower end of the receptor 56a. When the valve in the boundary to the receptor 56a is closed liquid-tightly, the treatment unit 56b is provided with a cover. When the valve in the boundary to the separation portion 57 is closed, the treatment unit 56b is provided with a bottom. The separation portion 57 is a cylindrical member having an inner wall which is liquid-lightly continuous from the inner wall of the treatment unit 56b and the valve is provided at its lower end. When this valve is closed liquid-tightly, the separation portion 57 is provided with a bottom. A separating member is fixed to an inside of the separation portion 57. When using the device 50, the container 58 is placed on the stage 54 beneath the lower part of the bottom of the separation portion 57. Here, the container 58 receives the specimen containing the separated subject cell 59 from the separation portion 57 and accommodates it therein. The treatment unit 56b may comprise a heating and/or thermo-regulating mechanism for a desired process such as digestion, a biochemical reaction and the like on the specimen contained therein. The separating member may be a component which separates and extracts a sample containing the subject cell from the specimen dropped from the treatment unit 56b, or may be a component which removes a contaminant from the specimen dropped from the treatment unit 56b. The separating member may spread along the surface intersecting the central axis of the separation portion 57 to be fixed over a circumference of the inner wall of the separation portion 57. The separating member may be, for example, a filter, a membrane and/or a magnet.

The sample containing the subject cell 59 separated with the separator 55 is accommodated in the container 58 placed on the stage 54. The container 58 which accommodates the subject cell is sent to the DNA introduction unit 52.

The DNA introduction unit 52 comprises a controller 60 and the introduction probe 61, which introduce a reporter vector to the subject cell accommodated in the container 58. The reporter vector may be introduced to the subject cell by a conventionally known method by itself. The controller 60 and the introduction probe 61 may only be devices which employ any introduction mechanism to realize the selected introduction method. For example, the DNA introduction unit 52 may be a device which performs electroporation. In that case, the controller 60 may be the DNA introducer and the introduction probe 61 may be an electrode probe, by which the electropolator may be constituted. Further, the DNA introduction unit 52 may comprise a nozzle (not shown) for supplying a reagent containing a reporter vector to the container 58. Furthermore, the DNA introduction unit 52 may comprise a liquid transfer system (not shown) for sending a reagent to the nozzle, and a reagent storage container (not shown) for supplying a reagent to the liquid transfer system.

Moreover, the DNA introduction unit 52 may function as an incubator which cultures the subject cell 59 in the container 58 under a predetermined culture condition over a predetermined time. The culture of the DNA introduction unit 52 may just be controlled by the controller 60.

In the DNA introduction unit 52, the container 58 containing the subject cell into which the reporter vector is introduced is, next, sent to the measurement unit 53.

The measurement unit 53 may comprise a black box 62 which covers a part of stage 54 from above and below, a light source 63 provided inside the black box 62, a window opened in a stage surface below the light source 63, an image pick-up unit 68 with an objective lens 67, installed under the window, a controller 65 communicating to the light source 63 and the image pick-up unit 68, and a display unit 64 and a storage unit 66, which are both connected to the controller 65. For example, the light source 63, the objective lens 67 and the image pick-up unit 68 of the measurement unit 53 may be installed to be contained in a microscope equipped with a CCD camera, a CMOS camera or the like. Moreover, the controller 65, the display unit 64 and the storage unit 66 may be a computer comprising these members.

FIG. 7 (B) shows an example of a device which differs from the device 50 shown in FIG. 7(A) only in the structure of the stage. The device 50 may include a cell separation unit 51, a DNA introduction unit 52, a measurement unit 53 and stages 54a, 54b and 54c disposed under these units, respectively. Thus, in the device 50 of FIG. 7(B), a stage is provided for each unit. A necessary item may be moved between the units, that is, between the stages manually or automatically using a robot arm or the like.

An example of the evaluation of the cell characteristics by the device 50 shown in FIG. 7(A) and (B) will be described. First, the container 58 is placed on the stage 54 below the separation portion 57 of the cell separation unit 51. Next, the specimen already subjected to the pretreatment of mincing, homogenization, etc. as needed is carried into the receptor 56a from the opening at the top. Simultaneously, a reagent required for the treatment with the treatment unit 56b is added. The reagent may be added from a closable reagent inlet opening formed in the treatment unit 56b. The receptor 56a is closed with its cover and the valve in the boundary to the treatment unit 56b is opened. According to the conditions set up in advance, the digestive treatment is carried out within the treatment unit 56b. The treatment unit 56b may comprise a thermo-regulator to create an environment which satisfies the predetermined conditions. Next, the valve in the boundary between the treatment unit 56b and the separation portion 57 is opened to send the specimen processed in the treatment unit 56b into the separation portion 57. While passing through the separating member of the separation portion 57, the contaminants contained in the specimen are removed. The sample containing the separated subject cell is sent to the container 58 and accommodated therein. Here, a reaction stop reagent for stopping the reaction progressing in the treatment unit 56b may be added to the specimen before passing through the separating member.

The reaction stop reagent may be accommodated in the separation portion 57 in advance, or such a reagent may be added through a closable reagent inlet opening made in a location of a wall surface of the separator 55.

With the sample containing the subject cell 59 accommodated therein, the container 58 is sent to the DNA introduction unit 52. A reagent containing a reporter vector is added to the container 58 through the nozzle of the DNA introduction unit 52. The introduction probe 61 is brought into contact with the sample in the container 58 and the reporter vector is introduced into the subject cell 59 under the control of the controller 60. Under the control of the controller 60, the subject cell 59 is cultured under a predetermined culture condition over a predetermined time.

Then, the container 58 is sent to the measurement unit 53. In the measurement unit 53, the quantity of the signal produced from the first reporter gene and the second reporter gene in the subject cell 59 accommodated in the container 58 is measured. All the procedures in the measurement unit 53 are controlled by the controller 65 according to a table in which the programs and a plurality of information pre-stored in the storage unit 66 are organized. For example, the light irradiation from the light source 63, the irradiation conditions such as the irradiation time and irradiation interval, the imaging with the image pick-up unit 68 and the image pick-up conditions, the image processing of images picked up, image processing, etc. are controlled by the measurement unit 53. Further, the evaluation of the cell characteristics is carried out by the controller 65 according to the program pre-stored in the storage unit 66 with reference to a table including the information which associate images and cell characteristics with each other.

After culturing the subject cell in the DNA introduction unit 52, the container 58 is sent to the measurement unit 53. Then, the light from the light source 63 is irradiated into the container 58. The light passing through the container 58 is concentrated by the objective lens 67, and with the light, a bright field image can be arbitrarily obtained with the image pick-up unit 68. Then, in the black box 62, the signal (for example, light emission) produced from the first reporter gene and the second reporter gene introduced to the subject cell is imaged by the image pick-up unit 68 through the objective lens 67. The light irradiation of the light source 63 is stopped and the light emission from the subject cell is collected with the objective lens 67, by which an image of the light emission is obtained with the image pick-up unit 68. Arbitrarily bright field images and light-emission images obtained by the image pick-up unit 68 are subjected to image processing by the controller 65, to be superimposed (merged) one on another and displayed on the display unit 64. At the same time or approximately the time of display of an image on the display unit 64, the controller 65 evaluates the characteristics of the subject cell based on the bright field image and light emission image with reference to the table stored in the storage unit 66. The result of the evaluation is displayed on the display unit 64 together with the merged image.

The above-provided example is directed to the case where light is emitted as a signal produced from the first reporter gene and the second reporter gene, but a signal other than light emission can be measured similarly by exchanging the sensing device which constitutes the image pick-up unit 68 as needed.

Moreover, the measurement unit 53 may further comprise a processor for performing computational operation, image processing, etc. or an analyzer for evaluation of characteristics. The processor and analyzer may perform the computational operation, image processing, and evaluation of characteristics carried out by the controller 65 in the above-discussed case.

In the above-described example the subject cell is cultured by the DNA introduction unit 52, but the subject cell may be cultured in the measurement unit 53, in which case, the device 50 should only comprise a component which unable cell culture. Or a subject cell may be cultured in both of the DNA introduction unit 52 and the measurement unit 53.

In order to control the motions of the units of the device 50, each unit may comprise a controller, or all the units included in the device 50 may be controlled by one controller, for example, the controller 65. Further, the container 58 placed on the stage 54 may be moved between units by a container transfer mechanism mounted in the stage 54. Such a mechanism may be, for example, the robot arm provided in the device 50 and controlled by the controller as to its movement. Or the conveyor belt may be attached to the stage 54.

Moreover, the reporter vector may be added to the container 58 at any stage before the container 58 is sent to a predetermined position of the DNA introduction unit 52. In that case, the device 50 should only further comprise a reporter vector addition mechanism at any location upstream the the DNA introduction unit 52.

The characteristics of a subject cell are evaluated by the above-described device. With this device, it is possible to evaluate the characteristics of the subject cell at higher precision. Further, it is possible to perform the test at a high throughput, the testing procedure is simple, thus making it possible to prevent mix-up of samples or contamination.

Moreover, the morphological feature of the subject cell may be acquired based on the bright field image, and the characteristics of the subject cell may be evaluated thereby together with the data from the reporter vector. The morphological feature may be, for example, the area of the subject cell, the ratio of the area of the nucleus to the area of the subject cell, the ratio of the minor axis to the major axis of the subject cell, the number of nuclei in the subject cell, or the feature of the marginal part of the subject cell, that is, for example, the contrast ratio of the marginal part, or the like. These features can be obtained by a noninvasive optical image pick-up method. For example, a hyper-spectrum imaging method, a phase-contrast microscope method, or a differential interferometry may be used to acquire these data.

For example, when performing morphologic observation by a bright field, it is preferable to irradiate light having a wavelength of a near-infrared region. For example, the light having a wavelength of a near-infrared region should preferably be near-infrared light having a wavelength of 700 nm to 750 nm or multiple-spectrum light having a wavelength of 350 nm to 1050 nm. For example, the light of a wavelength of the near-infrared region may be irradiated onto the subject cell to acquire the morphological data on the subject cell based on reflection or absorption of the light, for example, the reflectance or absorbance as an index.

For example, when the malignancy of a subject cell is high, there is a tendency that the cell form has a high aspect ratio, such as of a spindle shape. For example, the morphological data on such a subject cell as well may only be acquired in the measurement unit 53. In that case, the measurement unit 53 should just be controlled by the controller 65 according to the table in which the programs and information pre-stored in the storage unit 66 are organized. The table should just include information on the relationship between detectable signals from reporter vectors and cell characteristics and further information indicating the relationship between the morphological characteristics of the subject cell and the cell characteristics and the relationship between the detectable signals, cell characteristics and morphological characteristics. Moreover, for example, the light irradiation from the light source 63, the irradiation conditions such as the irradiation time and irradiation interval, the imaging with the image pick-up unit 68 and the image pick-up conditions, the image processing of images picked up, image processing, etc. are controlled by the measurement unit 53. Further, the evaluation of the cell characteristics may just be carried out by the controller 65 according to the program pre-stored in the storage unit 66 with reference to the table including the information which associate the morphological characteristics, information from a reporter vector, and cell characteristics with each other. Thus, by evaluating the cell characteristics of a subject cell from a plurality of sides including the promoter activity and the morphological characteristics, it is possible to obtain more accurate results.

### <Examples>

### Example 1: Evaluation of Structure of Dual Reporter Vector Using Breast Cancer Cell Strain

### (a) Production of Dual Reporter Vector

A reporter vector to which a promoter region (-2426 to +276bp, SEQ ID NO: 1) of a nicotinamidephosphoribosyl transferases (NAMPT) gene has been integrated was produced. The promoter region of the NAMPT gene amplified and adjusted by PCR using the human genome as the template. The PCR was performed using PrimeStar GXLDNA polymerase (TaKaRaBIO). The primer sequences used for the PCR are as follow.
Forward primer: 5'-GGGACGCGTCCATGTTGCCCAGGCTGGTCTCA-3'
Reverse primer: 5'-CGGCTCGAGAGCTCCCTGGCGCGGCTGCGAGGAA-3'

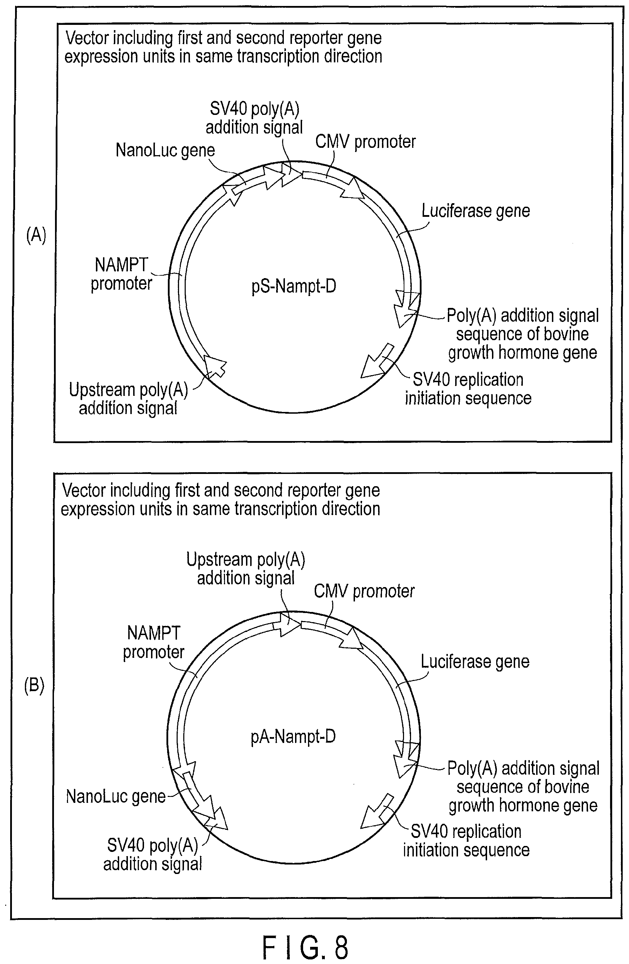

The promoter sequence (NAMPT promoter) of the NAMPT gene thus adjusted was integrated to an upstream of an Oplophorus gracilirostris-origin NanoLuc (registered trademark) gene, to produce a reporter gene expression unit comprising a NAMPT promoter sequence, a NanoLuc (Nluc) (registered trademark) gene and an SV40 transcription termination sequence, a reporter gene expression unit comprising a cytomegalovirus (CMV) promoter, a firefly-origin luciferase (Fluc, Firefly Luciferase) gene and a transcription termination sequence of a bovine growth hormone gene, and a self-replicating dual reporter vector containing SV40 replication initiation sequence on the same nucleic acid. As to the self-replicating dual reporter vector, two kinds were produced, in which the directions of reporter gene expression units differ from each other (that is, pS-NAMPT-D in FIG. 8 (A) and pA-NampD in FIG. 8 (B)). The self-replicating dual reporter vector was designed to comprise a first reporter gene expression unit including the promoter of the marker gene of the breast cancer cell strain as mentioned above (the unit including the NAMPT promoter sequence) and a second reporter gene expression unit a promoter exhibiting a constitutive activity (the unit including a CMV promoter).

### (b) Lipofection (Introduction of Reporter Vector to Cell)

0.5µg of the dual reporter vector was added to 100µL of Opti-MEM (Life Technologies). Then, 0.5µg of an enhancer reagent (Plus reagent of Life Technologies) was added and the resultant was incubated for 5 minutes at room temperature. To the solution, 1.5pL of Lipofectamine LTX (Life Technologies) was added and well suspended therein, and the resultant was incubated for 25 minutes at room temperature. 40µL of the solution was moved to a 48 well plate, and 200pL of a suspension of human breast cancer cell strain (high metastatic malignant tumor cell strain: MDA-MB-231) was added thereto, followed by gently mixing. Then, the cells were cultured in an incubator at 37°C in a 5%-CO₂ atmosphere.

### (c) Reporter Gene Assay

96 hours after the lipofection, the medium was removed from the 48 well plate and the cells were washed by PBS. Then, 150µL of a cytolysis liquid (Glo-Lysis Buffer, Promega) was added thereto and the resultant was let stand still for 30 minutes or more at -80°C to be frozen. When to be used, the cytolysis liquid was thawed at room temperature, and the solution was collected in a centrifuge tube and the cell residue was precipitated by centrifuge. The supernatant was collected in a new sample tube and 25µL of the supernatant was dispensed to a 96 well plate (Nunc). An equivalent amount of a Luciferase substrate solution (One-Glo Luciferase Assay System, Promega) or NanoLuc (registered trademark) substrate solution (Nano-Glo Luciferase Assay System, Promega) was added to the well, and the luminescent intensity was measured with a luminometer (Mithras LB940, Berthold). Thus, the reporter activity of Nluc (the activation of the NAMPT promoter) for evaluation of the malignancy of the oncocyte, and the reporter activation of Fluc (the activation of the CMV promoter) for the correction of amount of reporter DNA introduced for each cell were measured. The relative reporter activation (RLU) used for evaluation of the malignancy of the oncocyte was defined as a value obtained by dividing the activation value (luminescent intensity) of Nluc by the activation value (luminescent intensity) of Fluc.

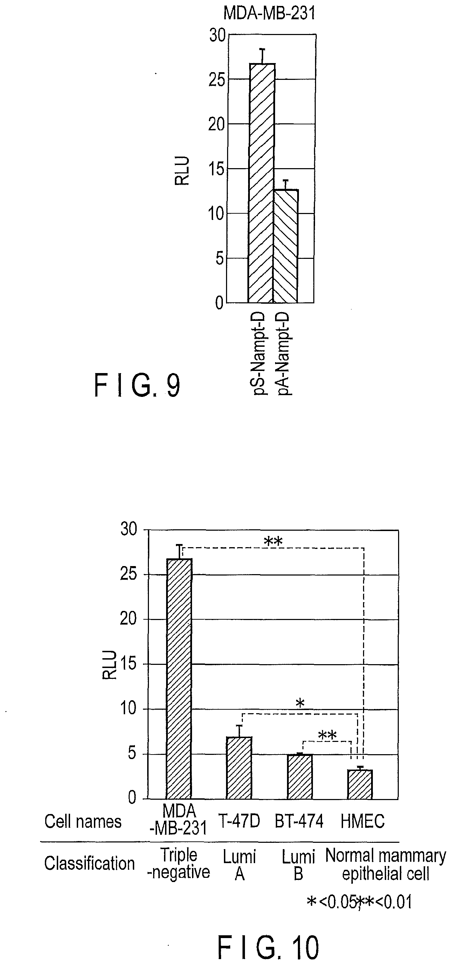

The results are shown in FIG. 9. The RLU in the high metastatic breast cancer cell strain with high malignancy exhibited a relative reporter activation higher in the pS-NAMPT-D to which two genetic expression units are incorporated in a forward direction, than in the pA-Namp-D to which two genetic expression units are integrated in a reverse direction. It was clarified from this result that the pS-NAMPT-D in which the two genetic expression units are directed in the forward direction has a dual reporter vector structure appropriate for evaluation of malignancy of oncocyte.

### Example 2: Evaluation of Malignancy/Non-malignancy of Breast Cancer Cell Strain Using Dual Reporter Vector: pS-NAMPT-D

By the method discussed in Example 1 (2), pS-NAMPT-D was introduced into a normal human mammary epithelial cell (HMEC) and three kinds of breast cancer cell strains (a high metastatic malignant oncocyte strain: MDA-MB-231 and low metastatic malignant oncocyte strains: T-47D and BT-474), and they were cultured. After 72 hours, a reporter gene assay was performed by the method discussed in Example 1 (3), and the relative reporter activity (RLU) was calculated.

The results are shown in FIG. 10. In all the breast cancer cell strains to which pS-NAMPT-D was introduced, a reporter activation significantly higher than that of HMEC was measured. The highest RLU was marked by the high metastatic malignant oncocyte strain MDA-MB-231. The RLU of the cell strain was about 7.8 times that of the HMEC. On the other hand, the RLUs of the low metastatic cell strains T-47D and BT-474 were 2.0 times and 1.4 times that of the HMEC, respectively. Using the reporter activity of pS-NAMPT-D as an index, all the malignant breast cancer cell strains could be detected, and a high RLU value was measured by the high metastatic and malignant oncocyte strain MDA-MB-231; therefore it was clarified that the pS-NAMPT-D has a dual reporter vector structure appropriate for evaluation of malignancy of oncocyte.

### Example 3. Evaluation of Structure of Self-replicating Dual Reporter Vector Using Breast Cancer Cell Strain and Evaluation of Malignancy/Non-malignancy of Breast Cancer Cell Strain

### (a) Construction of Reporter Vector

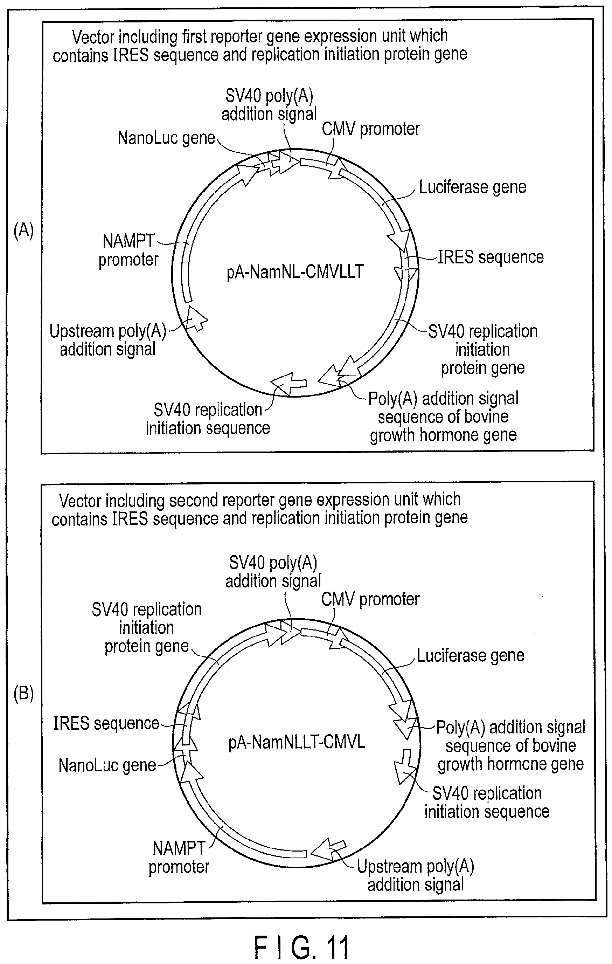

A self-replicating dual reporter vector (pAmp-NamNL-CMVLLT) was produced by insert a replication initiation protein (LT) gene of simian virus 40 (SV40) between a CMV promoter::luciferase gene, and a poly(A) addition signal of bovine growth hormone gene in pS-NAMPT-D (FIG. 11 (A)). Further, a self-replicating dual reporter vector (pAmp-NamNLLT-CMVL) was produced by inserting a replication initiation protein (LT) gene of SV40 between a NAMPT promoter::NanoLuc (registered trademark) gene and a poly(A) addition signal of SV40 in pS-NAMPT-D (FIG. 11 (B)).

### (b) Electroporation (Introduction of reporter vector to cell)

To ten kinds of malignant mammary gland-originated oncocyte strains (T-47D, MCF7, BT-474, UACC-812, SK-BR-3, HCC70, MDA-MB-468, BT-549, MDA-MB-231 and BT-20), one kind of benign mammary gland-originated tumor cell strain (MCF10A) and a normal human mammary epithelial cell (HMEC), the two kinds of self-replicating dual reporter vectors described in Section (a) above were integrated by electroporation. After pre-cultured cells were washed by PBS once, the cells were removed from the culture container using trypsin-EDTA, and a culture medium containing serum was added thereto to suspend the cells. Then, 50mL of the cell suspension was collected in a plastic centrifugal tube and the cells were precipitated by centrifuge. After that, the supernatant was removed, and Opti-MEM (Life Technologies) was added to suspend the cells. After precipitating the cells by centrifuge and removing the supernatant, the cell suspension was adjusted by adding Opti-MEM thereto to have a concentration of 1 × 10⁷ cell/mL. 100µL of the cell suspension was dispensed to a 1.5mL tube and 5.0µg of a reporter vector (pAmp-NamNL-CMVLLT or pAmp-NamNLLT-CMVL) was added thereto. After that, electroporation was performed using an electropolator (CUY EDIT II, Bex) and a cuvette electrode (2-mm width, Bex). Then, 1mL of a culture medium was added and gently mixed. After that, the cuvette electrode was set still in a 5%-CO₂ incubator. 5 minutes after, the cuvette electrode was removed from the incubator and the cell suspension was moved to a 24 well plate to which the culture medium was added in advance. Then, the resultant was cultured at 37°C in a 5%-CO₂ atmosphere.

### (c) Reporter Gene Assay

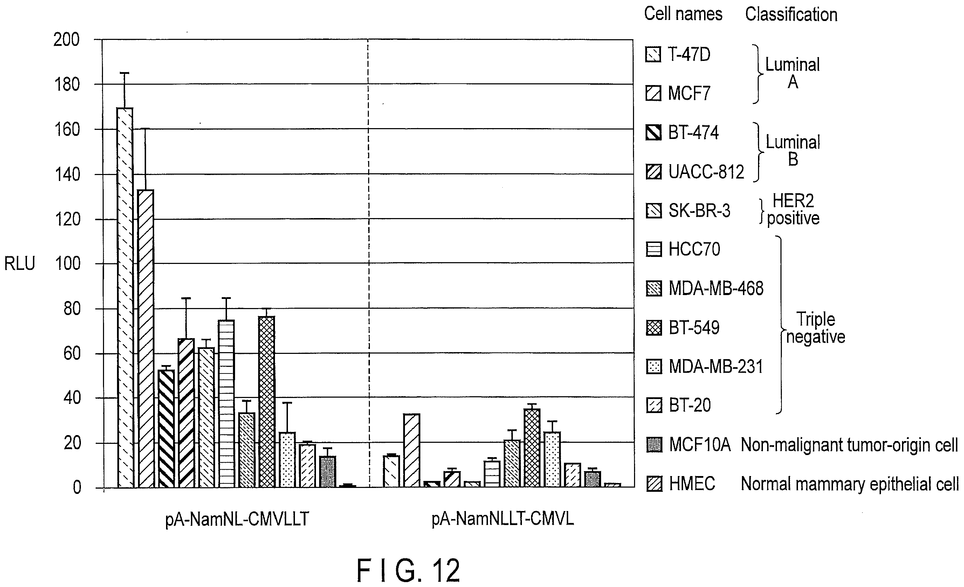

72 hours after the electroporation, a reporter gene assay was performed by the method discussed in Example 1 (3) to calculate the relative reporter activity (RLU). The results are shown in FIG. 12.

When pAmp-NamNL-CMVLLT was introduced, all the malignant oncocyte strains exhibited RLUs of 20 times that of the normal human mammary epithelial cell (HMEC). Further, even if compared with the RLUs of nonmalignant oncocyte strain MCF10A and malignant oncocyte strain, a RLU higher than that of MCF10A was exhibited in all the malignant breast cancer cells. On the other hand, when pAmp-NamNLLT-CMVL was integrated, a RLU lower than that of pAmp-NamNL-CMVLLT was exhibited. Further, with the same reporter vector, two kinds of malignant breast cancer cells exhibited RLUs comparable as that of HMEC, and three kinds of malignant breast cancer cells exhibited RLUs comparable with or lower than that of MCF10A. From this result, it was clarified that pAmp-NamNL-CMVLLT in which IRES sequence::replication initiation protein gene was inserted to a reporter gene expression unit including a reporter sequence exhibiting constitutive activity, which includes a CMV promoter exhibited a high signal value, and therefore has a reporter vector structure appropriate for evaluation of malignancy of a tumor with higher accuracy.

### Example 4. Microscopic Detection of Photogenesis of pAmp-NamNL-CMVLLT-introduced Breast Cancer Cell Strain

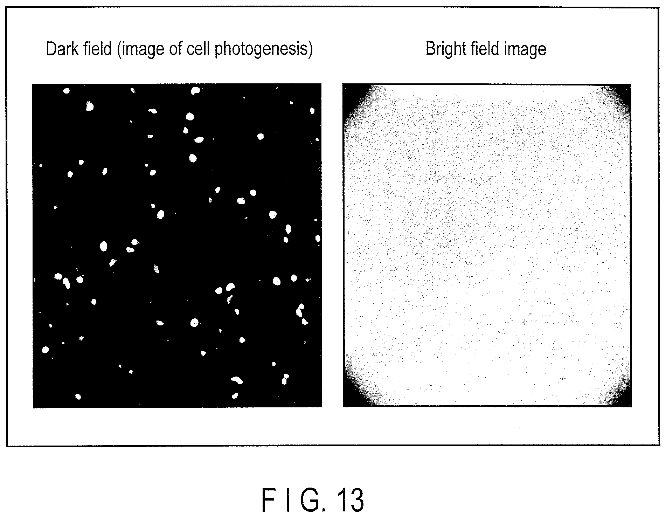

T-47D and HMEC were mixed at 1 : 1 or 1 : 3. To the thus mixed cells, pAmp-NamNL-CMVLLT was introduced by electroporation, the cells were cultured at 37°C in a 5%-CO₂ atmosphere. 72 hours after, photographs of the luminescent cells were taken using a photogenesis imaging system, LuminoView (LV200, Olympus). The results are shown in FIG. 13. The results indicated that cell photogenesis was observed in 97% of the pAmp-NamNL-CMVLLT-integrated T-47D. From this result, it was indicated that malignant breast cancer cell strains can be evaluated with pAmp-NamNL-CMVLLT.

It was clarified from the above-described experiment that the self-replicating dual reporter vectors of the embodiments are appropriate for evaluation of the characteristics of a subject cell with higher accuracy.

## Claims

1. A reporter vector for evaluating cell characteristics of a subject cell, **characterized by** comprising:
(1) a first reporter gene expression unit (13) including a NAMPT gene promoter sequence represented by SEQ ID N0.1 (10), a first reporter gene (11) operably linked to the NAMPT gene promoter sequence (10), and a first transcription termination sequence (12) existing downstream of the first reporter gene (11);
(2) a second reporter gene expression unit (25) including a promoter sequence exhibiting constitutive activity (20), a second reporter gene(21)operably linked to the promoter sequence exhibiting constitutive activity (20), a bicistronic expression sequence (22) existing downstream of the second reporter gene (21), a replication initiation protein gene (23) existing downstream of the bicistronic expression sequence (22), and a second transcription termination sequence (24) existing downstream of the bicistronic expression sequence (22); and
(3) a replication initiation sequence (30) for binding to a replication initiation protein synthesized from the replication initiation protein gene (23), thereby initiating replication of the reporter vector, wherein a first signal obtained from a first reporter protein expressed from the first reporter gene (11) and a second signal obtained from a second reporter protein expressed from the second reporter gene (21) are different from each other, and the transcription directions of the first reporter gene expression unit and the second reporter gene expression unit are the same.

2. The reporter vector of Claim 1, **characterized in that**
the first reporter gene (11) is expressed by activation of the NAMPT gene promoter sequence (10), and the second reporter gene (21) and the replication initiation protein gene (23) are expressed by activity of the promoter sequence exhibiting constitutive activity (20).

3. The reporter vector of Claim 1, **characterized in that**
the first reporter gene (11) and the second reporter gene (21) are each at least one of a blue fluorescent protein gene, a green fluorescent protein gene, a red fluorescent protein gene, a firefly luciferase gene, a renilla luciferase gene, a NanoLuc (registered trademark) luciferase gene, a chloramphenicol acetyltransferase gene, a nitric-monooxide synthetase gene, a xanthine-oxidase gene, a β-galactosidase gene or a heavy metal binding protein gene.

4. The reporter vector of Claim 1, **characterized in that**
the first transcription termination sequence (12) and the second transcription termination sequence (24) include a poly (A) sequence.

5. The reporter vector of Claim 4, **characterized in that**
the first transcription termination sequence (12) and the second transcription termination sequence (24) are poly (A) addition signal sequences.

6. The reporter vector of Claim 5, **characterized in that**
the first transcription termination sequence (12) and the second transcription termination sequence (24) are each at least one of a poly (A) addition signal sequence of simian virus 40, a poly (A) addition signal sequence of bovine growth hormone gene and an artificially synthesized poly (A) addition signal sequence.

7. The reporter vector of Claim 1, **characterized in that**
the promoter sequence exhibiting constitutive activity (20) is at least one of a cytomegalovirus promoter or a human herpesvirus thymidinekinase promoter.

8. The reporter vector of Claim 1, **characterized in that**
the bicistronic expression sequence (22) is an IRES sequence.

9. The reporter vector of Claim 8, **characterized in that** the IRES sequence is an IRES sequence of encephalomyocarditis virus.

10. The reporter vector of Claim 1, **characterized in that**
the replication initiation protein gene (23) is at least one of a wild type large T-antigen gene of simian virus 40, an EBNA-1 gene of Epstein-Barr virus or a large T-antigen gene of murine polyomavirus.

11. The reporter vector of Claim 1, **characterized in that** the replication initiation sequence (30) is a replication initiation sequence (30) of at least one of simian virus 40, Epstein-Barr virus or murine polyomavirus.

12. The reporter vector of Claim 1, **characterized in that**
the NAMPT gene promoter sequence (10) is a base sequence including a base sequence represented by SEQ ID NO: 1, the first and second transcription termination sequences (12, 24) are each a base sequence including a poly(A) addition signal sequence, the bicistronic expression sequence (22) is a base sequence including a sequence represented by SEQ ID NO: 7, the replication initiation protein gene (23) is a base sequence including a sequence represented by SEQ ID NO: 8, 9 or 10, and the replication initiation sequence (30) is a base sequence including a sequence represented by SEQ ID NO: 11.

13. An assay kit for evaluating cell characteristics of a subject cell containing a reporter vector and a carrier which supports the reporter vector, the reporter vector (1) **characterized by** comprising:
(1) a first reporter gene expression unit (13) including a NAMPT gene promoter sequence represented by SEQ ID NO.1 (10), a first reporter gene (11) operably linked to the NAMPT gene promoter sequence (10), and a first transcription termination sequence (12) existing downstream of the first reporter gene (11);
(2) a second reporter gene expression unit (25) including a promoter sequence exhibiting constitutive activity (20), a second reporter gene (21) operably linked to the promoter sequence exhibiting constitutive activity (20), a bicistronic expression sequence (22) existing in a downstream of the second reporter gene (21), a replication initiation protein gene (23) existing downstream of the bicistronic expression sequence (22), and a second transcription termination sequence (24) existing downstream of the bicistronic expressed sequence (22); and
(3) a replication initiation sequence (30) binding to a replication initiation protein synthesized from the replication initiation protein gene (23), thereby initiating replication of the reporter vector, wherein a first signal obtained from a first reporter protein expressed from the first reporter gene (11) and a second signal obtained from a second reporter protein expressed from the second reporter gene (21) are different from each other, and the transcription directions of the first reporter gene expression unit (13) and the second reporter gene expression unit (25) are the same.

14. A method of evaluating cell characteristics of a subject cell, **characterized by** comprising:
(a) introducing a reporter vector into the subject cell;
the reporter vector comprising:
(1) a first reporter gene expression unit (13) including a NAMPT gene promoter sequence represented by SEQ ID NO.1 (10), a first reporter gene (11) operably linked to the NAMPT gene promoter sequence, and a first transcription termination sequence (12) existing downstream of the first reporter gene (11);
(2) a second reporter gene expression unit (25) including a promoter sequence exhibiting constitutive activity (20), a second reporter gene (21) operably linked to the promoter sequence exhibiting constitutive activity (20), a bicistronic expression sequence (22) existing downstream of the second reporter gene (21), a replication initiation protein gene (23) existing downstream of the bicistronic expression sequence, and a second transcription termination sequence (24) existing downstream of the bicistronic expression sequence (22); and
(3) a replication initiation sequence (30) binding to a replication initiation protein synthesized from the replication initiation protein gene (23), thereby initiating replication of the reporter vector,
wherein
a first signal obtained from a first reporter protein expressed from the first reporter gene (11) and a second signal obtained from a second reporter protein expressed from the second reporter gene (21) are different from each other, and
transcription directions of the first reporter gene expression unit (13) and the second reporter gene expression unit (25) are the same,
(b) causing the reporter vector to replicate and expressing the first reporter gene (11) and the second reporter gene (21);
(c) detecting a first signal from the first reporter gene (11) and a second signal from the second reporter gene (21); and
(d) evaluating the cell characteristics of the subject cell by comparing the first signal and the second signal, wherein the cell characteristics are whether or not there are indexes which indicate a sign of a disease with a tumor, onset of a disease with a tumor, a grade of progression of the onset of a disease with a tumor and/or severity of a disease with a tumor.

15. The method of Claim 14, **characterized in that**
the subject cell is of an epithelial tumor, non-epithelial tumor, hemopoietic tissue tumor, fetal tumor, or any combination thereof.

## Patentansprüche

1. Reporter-Vektor zum Evaluieren von Zell-Charakteristika einer Zielzelle (subject cell), charakterisiert durch Umfassen von:
(1) eine erste Reportergen-Expressionseinheit (13), einschließend eine NAMPT-Gen-Promotorsequenz, repräsentiert von SEQ ID NO. 1 (10), einem ersten Reportergen (11), funktionell mit der NAMPT-Gen-Promotorsequenz (10) verknüpft, und eine erste Transkriptions-Terminationssequenz (12), welche downstream des ersten Reportergens (11) existiert;
(2) eine zweite Reportergen-Expressionseinheit (25), einschließend eine Promotorsequenz, welche konstitutive Aktivität aufweist (20), ein zweites Reportergen, funktionell mit der Promotorsequenz, welche konstitutive Aktivität aufweist (20), verknüpft, eine bicistronische Expressionssequenz (22), welche downstream des zweiten Reportergens (21) existiert, ein Replikationsinitiationsprotein-Gen (23), welches downstream der bicistronischen Expressionssequenz (22) existiert, und eine zweite Transkriptions-Terminationssequenz (24), welche downstream der bicistronischen Expressionssequenz (22) existiert; und
(3) eine Replikationsinitiationsequenz (30) für Binden an ein Replikationsinitiationsprotein, vom Replikationsinitiationsprotein-Gen (23) synthetisiert, dadurch Initiieren der Replikation des Reporter-Vektors, wobei ein erstes Signal, erhalten von einem von dem ersten Reportergen (11) exprimierten ersten Reporterprotein, und ein zweites Signal, erhalten von dem einem von dem zweiten Reportergen (21) exprimierten zweiten Reporterprotein, unterschiedlich voneinander sind, und die Transkriptionsrichtungen der ersten Reportergen-Expressionseinheit und der zweiten Reportergen-Expressionseinheit gleich sind.

2. Reporter-Vektor gemäß Anspruch 1, charakterisiert dadurch, dass das erste Reportergen (11) durch Aktivierung der NAMPT-Gen-Promotorsequenz (10) exprimiert wird, und das zweite Reportergen (21) und das Replikationsinitiationsprotein-Gen (23) durch Aktivität der Promotorsequenz, welche konstitutive Aktivität aufweist (20), exprimiert werden.

3. Reporter-Vektor gemäß Anspruch 1, charakterisiert dadurch, dass das erste Reportergen (11) und das zweite Reportergen (21) jeweils mindestens eines aus einem blau fluoreszenten Protein-Gen, einem grün fluoreszenten Protein-Gen, einem roten fluoreszenten Protein-Gen, einem Glühwürmchen-Luciferase-Gen, einem Renilla-Luciferase-Gen, einem NanoLuc (eingetragenes Markenzeichen)-Luciferase-Gen, einem Chloramphenicol-Acetyltransferase-Gen, einem Stickstoffmonoxid-Synthetase-Gen, einem Xanthin-Oxidase-Gen, einem β-Galactosidase-Gen oder einem Schwermetall-bindendem-Protein-Gen sind.

4. Reporter-Vektor gemäß Anspruch 1, charakterisiert dadurch, dass die erste Transkriptions-Terminationssequenz (12) und die zweite Transkriptions-Terminationssequenz (24) eine poly(A)-Sequenz einschließen.

5. Reporter-Vektor gemäß Anspruch 4, charakterisiert dadurch, dass die erste Transkriptions-Terminationssequenz (12) und die zweite Transkriptions-Terminationssequenz (24) poly(A)-Additions-Signalsequenzen sind.

6. Reporter-Vektor gemäß Anspruch 5, charakterisiert dadurch, dass die erste Transkriptions-Terminationssequenz (12) und die zweite Transkriptions-Terminationssequenz (24) jeweils mindestens eines einer poly(A)-Additions-Signalsequenz des Simian-Virus 40, einer poly(A)-Additions-Signalsequenz des bovinen Wachstumshormon-Gen und einer künstlich synthetisierten poly(A)-Additions-Signalsequenz sind.

7. Reporter-Vektor gemäß Anspruch 1, charakterisiert dadurch, dass die Promotorsequenz, welche konstitutive Aktivität aufweist (20) mindestes eine eines Cytomegalovirus-Promotor oder eines humanen Herpesvirus Thymidinkinase-Promotors ist.

8. Reporter-Vektor gemäß Anspruch 1, charakterisiert dadurch, dass die bicistronische Expressionssequenz (22) eine IRES-Sequenz ist.

9. Reporter-Vektor gemäß Anspruch 8, charakterisiert dadurch, dass die IRES-Sequenz eine IRES-Sequenz des Encephalomyocarditis-Virus ist.

10. Reporter-Vektor gemäß Anspruch 1, charakterisiert dadurch, dass das Replikationsinitiationsprotein-Gen (23) mindestens eines aus einem Wildtyp großen T-Antigen des Simian-Virus 40, einem EBNA-1-Gen des Epstein-Barr-Virus oder einem großen T-Antigen des murinen Polyomavirus ist.

11. Reporter-Vektor gemäß Anspruch 1, charakterisiert dadurch, dass die Replikationsinitiationssequenz (30) eine Replikationsinitiationssequenz (30) von mindestens einem aus Simian-Virus 40, Epstein-Barr-Virus oder murinem Polyomavirus ist.

12. Reporter-Vektor gemäß Anspruch 1, charakterisiert dadurch, dass die NAMPT-Gen-Promotorsequenz (10) eine Basen-Sequenz, welche eine von SEQ ID NO: 1 repräsentierte Basen-Sequenz einschließt, ist, die erste und zweite Transkriptions-Terminationssequenz(en) (12, 24) jeweils eine Basensequenz, welche eine poly(A)-Additions-Signalsequenz einschließt, ist/sind, die bicistronische Expressions-Sequenz eine Basen-Sequenz, welche eine von SEQ ID NO: 7 repräsentierte Sequenz einschließt, ist, das Replikationsinitiationsprotein-Gen (23) eine Basen-Sequenz, welche eine von SEQ ID NO: 8, 9 oder 10 repräsentierte Sequenz einschließt, ist, die Replikationsinitiationssequenz eine Basen-Sequenz, welche eine von SEQ ID NO: 11 repräsentierte Sequenz einschließt, ist.

13. Assay-Kit zum Evaluieren von Zell-Charakteristika einer Zielzelle, welche einen Reporter-Vektor und einen Trägerstoff, welchen den Reporter-Vektor unterstützt, beinhaltet, der Reporter-Vektor (1) charakterisiert durch Umfassen von:
(1) eine erste Reportergen-Expressionseinheit (13), einschließend eine NAMPT-Gen-Promotorsequenz, repräsentiert von SEQ ID NO. 1 (10), einem ersten Reportergen (11), funktionell mit der NAMPT-Gen-Promotorsequenz (10) verknüpft, und eine erste Transkriptions-Terminationssequenz (12), welche downstream des ersten Reportergens (11) existiert;
(2) eine zweite Reportergen-Expressionseinheit (25), einschließend eine Promotorsequenz, welche konstitutive Aktivität aufweist (20), ein zweites Reportergen, funktionell mit der Promotorsequenz, welche konstitutive Aktivität aufweist (20), verknüpft, eine bicistronische Expressionssequenz (22), welche downstream des zweiten Reportergens (21) existiert, ein Replikationsinitiationsprotein-Gen (23), welches downstream der bicistronischen Expressionssequenz (22) existiert, und eine zweite Transkriptions-Terminationssequenz (24), welche downstream der bicistronischen Expressionssequenz (22) existiert; und
(3) eine Replikationsinitiationsequenz (30) für Binden an ein Replikationsinitiationsprotein, vom Replikationsinitiationsprotein-Gen (23) synthetisiert, dadurch Initiieren der Replikation des Reporter-Vektors, wobei ein erstes Signal, erhalten von einem von dem ersten Reportergen (11) exprimierten ersten Reporterprotein, und ein zweites Signal, erhalten von einem von dem zweiten Reportergen (21) exprimierten zweiten Reporterprotein, unterschiedlich voneinander sind, und die Transkriptionsrichtungen der ersten Reportergen-Expressionseinheit und der zweiten Reportergen-Expressionseinheit gleich sind.

14. Verfahren zum Evaluieren von Zell-Charakteristika einer Zielzelle, charakterisiert durch Umfassen von:
(a) Einbringen eines Reporter-Vektors in die Zielzelle;
der Reporter-Vektor umfassend:
(1) eine erste Reportergen-Expressionseinheit (13), einschließend eine NAMPT-Gen-Promotorsequenz, repräsentiert von SEQ ID NO. 1 (10), einem ersten Reportergen (11), funktionell mit der NAMPT-Gen-Promotorsequenz (10) verknüpft, und eine erste Transkriptions-Terminationssequenz (12), welche downstream des ersten Reportergens (11) existiert;
(2) eine zweite Reportergen-Expressionseinheit (25), einschließend eine Promotorsequenz, welche konstitutive Aktivität aufweist (20), ein zweites Reportergen, funktionell mit der Promotorsequenz, welche konstitutive Aktivität aufweist (20), verknüpft, eine bicistronische Expressionssequenz (22), welche downstream des zweiten Reportergens (21) existiert, ein Replikationsinitiationsprotein-Gen (23), welches downstream der bicistronischen Expressionssequenz (22) existiert, und eine zweite Transkriptions-Terminationssequenz (24), welche downstream der bicistronischen Expressionssequenz (22) existiert; und
(3) eine Replikationsinitiationsequenz (30) für Binden an ein Replikationsinitiationsprotein, vom Replikationsinitiationsprotein-Gen (23) synthetisiert, dadurch Initiieren der Replikation des Reporter-Vektors, wobei ein erstes Signal, erhalten von einem von dem ersten Reportergen (11) exprimierten ersten Reporterprotein, und ein zweites Signal, erhalten von einem von dem zweiten Reportergen (21) exprimierten zweiten Reporterprotein, unterschiedlich voneinander sind, und die Transkriptionsrichtungen der ersten Reportergen-Expressionseinheit und der zweiten Reportergen-Expressionseinheit gleich sind,
(b) Bewirken des Reporter-Vektors zu replizieren und das erste Reportergen (11) und das zweite Reportergen (21) zu exprimieren;
(c) Detektieren eines ersten Signals von dem ersten Reportergen (11) und eines zweiten Signals von dem zweiten Reportergen (21); und
(d) Evaluieren der Zell-Charakteristika der Zielzelle durch Vergleichen des ersten Signals und des zweiten Signals, wobei die Zell-Charakteristika sind, ob oder ob nicht Indices, welche ein Zeichen einer Krankheit mit einem Tumor, Ausbruch einer Krankheit mit einem Tumor, ein Grad des Verlaufs des Ausbruchs einer Krankheit mit einem Tumor und/oder den Schweregrad einer Krankheit mit einem Tumor anzeigen, vorhanden sind.

15. Verfahren gemäß Anspruch 14, charakterisiert dadurch, dass die Zielzelle von einem epithelialen Tumor, nichtepithelialen Tumor, hämatopoetischen Gewebe-Tumor, fötalen Tumor oder irgendeiner Kombination davon ist.

## Revendications

1. Vecteur rapporteur pour l'évaluation des caractéristiques cellulaires d'une cellule d'un sujet, **caractérisé par** le fait de comprendre:
(1) une première unité d'expression de gène rapporteur (13) incluant une séquence de promoteur de gène NAMPT représentée par la SEQ ID NO.1 (10), un premier gène rapporteur (11) lié de manière fonctionnelle à la séquence de promoteur de gène NAMPT (10) et une première séquence de terminaison de transcription (12) existant en aval du premier gène rapporteur (11);
(2) une deuxième unité d'expression de gène rapporteur (25) incluant une séquence de promoteur affichant une activité constitutive (20), un deuxième gène rapporteur (21) lié de manière fonctionnelle à la séquence de promoteur affichant une activité constitutive (20), une séquence d'expression bicistronique (22) existant en aval du deuxième gène rapporteur (21), un gène de protéine d'initiation de réplication (23) existant en aval de la séquence d'expression bicistronique (22) et une deuxième séquence de terminaison de transcription (24) existant en aval de la séquence d'expression bicistronique (22); et
(3) une séquence d'initiation de réplication (30) pour une liaison à une protéine d'initiation de réplication synthétisée à partir du gène de protéine d'initiation de réplication (23), initiant ainsi la réplication du vecteur rapporteur, où un premier signal obtenu à partir d'une première protéine rapporteuse exprimée à partir du premier gène rapporteur (11) et un deuxième signal obtenu à partir d'une deuxième protéine rapporteuse exprimée à partir du deuxième gène rapporteur (21) sont différents l'un de l'autre et les directions de transcription de la première unité d'expression de gène rapporteur et de la deuxième unité d'expression de gène rapporteur sont les mêmes.

2. Vecteur rapporteur selon la revendication 1, **caractérisé en ce que** le premier gène rapporteur (11) est exprimé par l'activation de la séquence de promoteur de gène NAMPT (10) et le deuxième gène rapporteur (21) et le gène de protéine d'initiation de réplication (23) sont exprimés par l'activité de la séquence de promoteur affichant une activité constitutive (20).

3. Vecteur rapporteur selon la revendication 1, **caractérisé en ce que** le premier gène rapporteur (11) et le deuxième gène rapporteur (21) sont chacun au moins un parmi un gène de protéine fluorescente bleue, un gène de protéine fluorescente verte, un gène de protéine fluorescente rouge, un gène de luciférase de luciole, un gène de luciférase de Renilla, un gène de luciférase NanoLuc (marque de fabrique déposée), un gène de chloramphénicol acétyltransférase, un gène de monooxyde nitrique synthétase, un gène de xanthine-oxydase, un gène de β-galactosidase ou un gène de protéine de liaison de métaux lourds.

4. Vecteur rapporteur selon la revendication 1, **caractérisé en ce que** la première séquence de terminaison de transcription (12) et la deuxième séquence de terminaison de transcription (24) incluent une séquence de poly (A).

5. Vecteur rapporteur selon la revendication 4, **caractérisé en ce que** la première séquence de terminaison de transcription (12) et la deuxième séquence de terminaison de transcription (24) sont des séquences de signal d'addition de poly (A).

6. Vecteur rapporteur selon la revendication 5, **caractérisé en ce que** la première séquence de terminaison de transcription (12) et la deuxième séquence de terminaison de transcription (24) sont chacune au moins une parmi une séquence de signal d'addition de poly (A) de virus simien 40, une séquence de signal d'addition de poly (A) de gène d'hormone de croissance bovin et une séquence de signal d'addition de poly (A) synthétisée artificiellement.

7. Vecteur rapporteur selon la revendication 1, **caractérisé en ce que** la séquence de promoteur affichant une activité constitutive (20) est au moins un parmi un promoteur de cytomégalovirus ou un promoteur de thymidine kinase de virus de l'herpès humain.

8. Vecteur rapporteur selon la revendication 1, **caractérisé en ce que** la séquence d'expression bicistronique (22) est une séquence IRES.

9. Vecteur rapporteur selon la revendication 8, **caractérisé en ce que** la séquence IRES est une séquence IRES de virus d'encéphalomyocardite.

10. Vecteur rapporteur selon la revendication 1, **caractérisé en ce que** le gène de protéine d'initiation de réplication (23) est au moins un parmi un gène d'antigène T de grande taille de type sauvage de virus simien 40, un gène EBNA-1 de virus d'Epstein-Barr ou un gène d'antigène T de grande taille de polyomavirus murin.

11. Vecteur rapporteur selon la revendication 1, **caractérisé en ce que** la séquence d'initiation de réplication (30) est une séquence d'initiation de réplication (30) d'au moins un parmi un virus simien 40, un virus d'Epstein-Barr ou un polyomavirus murin.

12. Vecteur rapporteur selon la revendication 1, **caractérisé en ce que** la séquence de promoteur de gène NAMPT (10) est une séquence de base incluant une séquence de base représentée par la SEQ ID NO: 1, les première et deuxième séquences de terminaison de transcription (12, 24) sont chacune une séquence de base incluant une séquence de signal d'addition de poly (A), la séquence d'expression bicistronique (22) est une séquence de base incluant une séquence représentée par la SEQ ID NO: 7, le gène de protéine d'initiation de réplication (23) est une séquence de base incluant une séquence représentée par la SEQ ID NO: 8, 9 ou 10 et la séquence d'initiation de réplication (30) est une séquence de base incluant une séquence représentée par la SEQ ID NO: 11.

13. Kit de dosage pour l'évaluation des caractéristiques cellulaires d'une cellule d'un sujet contenant un vecteur rapporteur et un véhicule qui supporte le vecteur rapporteur, le vecteur rapporteur (1) étant **caractérisé par** le fait de comprendre:
(1) une première unité d'expression de gène rapporteur (13) incluant une séquence de promoteur de gène NAMPT représentée par la SEQ ID NO.1 (10), un premier gène rapporteur (11) lié de manière fonctionnelle à la séquence de promoteur de gène NAMPT (10) et une première séquence de terminaison de transcription (12) existant en aval du premier gène rapporteur (11);
(2) une deuxième unité d'expression de gène rapporteur (25) incluant une séquence de promoteur affichant une activité constitutive (20), un deuxième gène rapporteur (21) lié de manière fonctionnelle à la séquence de promoteur affichant une activité constitutive (20), une séquence d'expression bicistronique (22) existant en aval du deuxième gène rapporteur (21), un gène de protéine d'initiation de réplication (23) existant en aval de la séquence d'expression bicistronique (22) et une deuxième séquence de terminaison de transcription (24) existant en aval de la séquence d'expression bicistronique (22); et
(3) une séquence d'initiation de réplication (30) se liant à une protéine d'initiation de réplication synthétisée à partir du gène de protéine d'initiation de réplication (23), initiant ainsi la réplication du vecteur rapporteur, où un premier signal obtenu à partir d'une première protéine rapporteuse exprimée à partir du premier gène rapporteur (11) et un deuxième signal obtenu à partir d'une deuxième protéine rapporteuse exprimée à partir du deuxième gène rapporteur (21) sont différents l'un de l'autre et les directions de transcription de la première unité d'expression de gène rapporteur (13) et de la deuxième unité d'expression de gène rapporteur (25) sont les mêmes.

14. Méthode pour l'évaluation des caractéristiques cellulaires d'une cellule d'un sujet, **caractérisée par** le fait de consister à:
(a) introduire un vecteur rapporteur dans la cellule du sujet;
le vecteur rapporteur comprenant:
(1) une première unité d'expression de gène rapporteur (13) incluant une séquence de promoteur de gène NAMPT représentée par la SEQ ID NO.1 (10), un premier gène rapporteur (11) lié de manière fonctionnelle à la séquence de promoteur de gène NAMPT et une première séquence de terminaison de transcription (12) existant en aval du premier gène rapporteur (11);
(2) une deuxième unité d'expression de gène rapporteur (25) incluant une séquence de promoteur affichant une activité constitutive (20), un deuxième gène rapporteur (21) lié de manière fonctionnelle à la séquence de promoteur affichant une activité constitutive (20), une séquence d'expression bicistronique (22) existant en aval du deuxième gène rapporteur (21), un gène de protéine d'initiation de réplication (23) existant en aval de la séquence d'expression bicistronique et une deuxième séquence de terminaison de transcription (24) existant en aval de la séquence d'expression bicistronique (22); et
(3) une séquence d'initiation de réplication (30) se liant à une protéine d'initiation de réplication synthétisée à partir du gène de protéine d'initiation de réplication (23), initiant ainsi la réplication du vecteur rapporteur,
où
un premier signal obtenu à partir d'une première protéine rapporteuse exprimée à partir du premier gène rapporteur (11) et un deuxième signal obtenu à partir d'une deuxième protéine rapporteuse exprimée à partir du deuxième gène rapporteur (21) sont différents l'un de l'autre et
les directions de transcription de la première unité d'expression de gène rapporteur (13) et de la deuxième unité d'expression de gène rapporteur (25) sont les mêmes,
(b) faire répliquer le vecteur rapporteur et exprimer le premier gène rapporteur (11) et le deuxième gène rapporteur (21);
(c) détecter un premier signal provenant du premier gène rapporteur (11) et un deuxième signal provenant du deuxième gène rapporteur (21); et
(d) évaluer les caractéristiques cellulaires de la cellule du sujet en comparant le premier signal et le deuxième signal, où les caractéristiques cellulaires sont s'il y a ou non des indices qui indiquent un signe d'une maladie avec une tumeur, l'attaque d'une maladie avec une tumeur, un degré de progression de l'attaque d'une maladie avec une tumeur et/ou la gravité d'une maladie avec une tumeur.

15. Méthode selon la revendication 14, **caractérisée en ce que** la cellule du sujet est une parmi une tumeur épithéliale, une tumeur non épithéliale, une tumeur de tissu hémopoïétique, une tumeur foetale ou toute combinaison de celles-ci.
